# EUROPEAN PATENT APPLICATION

(11) **EP 2 821 071 A1**
(43) Date of publication of application: **07.01.2015**
(21) Application number: 13382269.2
(22) Date of filing: 04.07.2013
(51) Int. Cl.: A61K 31/416, A61K 31/4535, A61K 31/506, A61K 31/565, A61K 45/06, A61P 35/00

(54) **Compounds for breast cancer treatment**

(71) Applicant: Institut d'Investigació Biomèdica de Bellvitge (IDIBELL), 08908 Hospitalet de Llobregat (ES); Institut Català d'Oncologia (ICO), 08908 L'Hospitalet de Llobregat (ES)
(72) Inventor: Urruticoechea Ribate, Ander, E-08908 L'Hospitalet de Llobregat (ES); Aguilar Calafell, Helena, E-08908 L'Hospitalet de Llobregat (ES); Genestar Pujana, Miguel Angel, E-08908 L'Hospitalet de Llobregat (ES)
(74) Representative: ABG Patentes, S.L.

(57) **Abstract**

The present invention is related to 1-benzyl-3-(substituted aryl) condensed pyrazoles and compositions thereof for their use in the treatment of breast cancer, in particular in the treatment of breast cancer resistant to endocrine therapy. The compounds preferably are YC-1 or BAY 41-2272. Optionally, estrogen receptor antagonists, selective estrogen receptor modulators, aromatase inhibitors, ErbB3 inhibitors, EGFR inhibitors and/or mTOR inhibitors are coadministered.

## Description

### FIELD OF THE INVENTION

The present invention belongs to the field of cancer treatment and is related to compounds for their use in the treatment of cancer, in particular in the treatment of breast cancer resistant to endocrine therapy.

### BACKGROUND OF THE INVENTION

Breast cancer is the second most common type of cancer worldwide (10.4%, after lung cancer) and the fifth most common cause of cancer-induced death (after lung cancer, stomach cancer, liver cancer, and colon cancer). Breast cancer is the most common cause of cancer-induced death among women worldwide. In 2005, breast cancer caused 502,000 deaths all over the world (7% of cancer-induced deaths; almost 1% of all deaths). The number of cases worldwide has significantly increased since the 1970s, a phenomenon which can partially be attributed to modem Western lifestyles. Women in North America have the highest incidence of breast cancer in the world.

Since the breast is made up of identical tissues in men and women, breast cancer also occurs in men. The incidence of breast cancer in men is approximately 100 times less than in women, but it is considered that men with breast cancer statistically have the same survival rates as women.

Breast cancer is staged according to the TNM (tumor-node-metastasis) system. The prognosis is closely related to the results of the staging, and the staging is also used to assign patients to treatments both in clinical trials and in medical practice. The information for staging is as follows:
- TX: The primary tumor cannot be evaluated. T0: There is no evidence of tumor. Tis: Carcinoma in situ, no invasion. T1: The tumor is 2 cm or less across. T2: The tumor is more than 2 cm but less than 5 cm across. T3: The tumor is more than 5 cm across. T4: Tumor of any size growing into the chest wall or skin, or inflammatory breast cancer.
- NX: The nearby lymph nodes cannot be evaluated. N0: The cancer has not spread to regional lymph nodes. N1: The cancer has spread to 1 to 3 underarm lymph nodes or to an internal mammary lymph node. N2: The cancer has spread to 4 to 9 underarm lymph nodes or to multiple internal mammary lymph nodes. N3: Any of the following:
   - The cancer has spread to 10 or more underarm lymph nodes, or the cancer has spread to the lymph nodes under the clavicle, or the cancer has spread to the lymph nodes above the clavicle or the cancer affects underarm lymph nodes and has spread to internal mammary lymph nodes, or the cancer affects 4 or more underarm lymph nodes, and small amounts of cancer are found in the internal mammary lymph nodes or in sentinel lymph node biopsy.
- MX: The presence of distant spread (metastasis) cannot be evaluated. M0: No distant spread is found. M1: Spread to distant organs is present, these organs not including the lymph node above the clavicle.

The principal pillar of breast cancer treatment is surgery when the tumor is localized, with possible adjuvant hormone therapy (with tamoxifen or an aromatase inhibitor), chemotherapy, and/or radiotherapy. Over two thirds of breast cancer cases in the general population present tumors that appreciably express ERα. Two major strategies mediate the efficacy of endocrine therapies directed against cell proliferation and survival mediated by ERα. Drugs directed at ERα, mainly tamoxifen and fulvestrant (ICI-182,780), prevent its binding to estradiol or promote the degradation of the receptor, respectively, thereby blocking canonical ERα-dependent transcriptional regulation. In contrast, the activity of aromatase inhibitors (AIs, e.g. anastrozole, letrozole, and exemestane), which are the most effective treatment in postmenopausal women (the largest group of patients), is based on deprivation of estrogen production.

Despite the documented benefits of ER-targeted therapy in breast cancer, it is known that not all patients who have estrogen receptor (ER) or progesterone receptor (PgR) expressing tumors respond to endocrine manipulation (*de novo* resistance) and a substantial number of patients who do respond will develop disease progression or recurrence while on therapy (acquired resistance). This acquired resistance occurs both in the form of a substantial rate of tumor relapse after excision, during adjuvant treatment (e.g. advanced disease), and as a near-universal event when tumors cannot be excised.

While some of the predictors of endocrine therapy failure are clinical factors, such as poor performance status and other indicators of bulky disease, molecular features other than the ER itself must play a role in determining the degree of benefit from endocrine therapy. Endocrine therapy failures and disease relapses still occur even in clinically disease-free patients in the adjuvant setting who have excellent performance status.

Preclinical models have shown that an adaptive up-regulation of growth factor signaling is associated with acquired resistance to endocrine therapies whereas overexpression of HER2 in ER positive breast cancer is responsible for *de novo* resistance to tamoxifen and aromatase inhibitors (Pancholi AE et al. 2008 Endocr Relat Cancer 15: 985-1002). Recent studies on ER biology have highlighted the fundamental role of the intimate cross talk between the ER and HER2/growth factor signaling pathways on resistance development to hormone therapies (Knowlden JM et al. 2003 Endocrinology 144:1032-1044). It has been suggested that overactive growth factor receptor signaling through multiple intracellular pathways may contribute to the evolution of an endocrine resistance phenotype.

Current knowledge on the mechanisms of acquired resistance supports a model in which the crosstalk between ERα (non-canonical function) and growth factor signaling pathways plays a fundamental role. In this regard, clinical trials that assess the combination of AIs and signaling pathway inhibitors-principally everolimus (Afinitor, Novartis) for inhibition of the mammalian target of rapamycin (mTOR) complex 1 (mTORC1) have shown promising results. Nonetheless, there is not a clear biomarker from the mTORC1 pathway that predicts treatment response. Complementarily, fibroblast and epidermal growth factor signaling pathway components have been suggested to play an important role in the acquisition of resistance. In this scenario, it remains to be defined which signaling component(s) might be most critical, how they might be exploited for the development of targeted therapies that could prevent or overcome resistance, and how they could eventually be used as predictive biomarkers.

Despite the efforts made to date, there still exists a long-felt and continuing need in the art for novel compounds and/or therapies useful in breast cancer treatment, particularly in those breast cancer patients who become irresponsive to hormone therapy based on aromatase inhibitors, estrogen receptor antagonists or selective estrogen receptor modulators.

### SUMMARY OF THE INVENTION

The invention relates to a compound of formula (I) 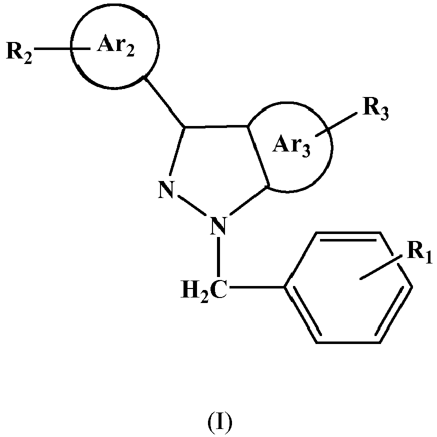 wherein
R₁ represents H, C₁₋₃ alkyl, halogen, or -OR, in which R represents H or C₁₋₃ alkyl; 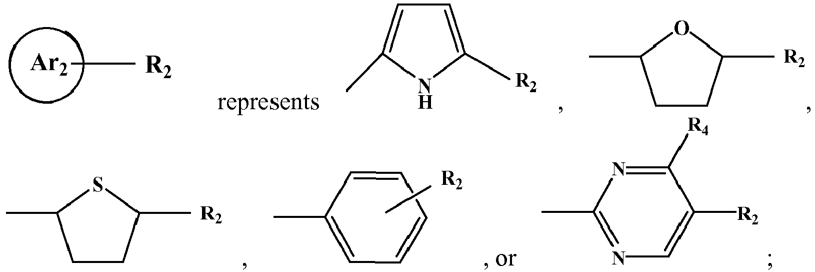 R₂ represents -COOR, -CH₂OR, H, C₁₋₃ alkyl, C₃₋₇ cycloalkyl, or halogen, in which R represents H or C₁₋₃ alkyl;
R₄ represents H, -NH₂, or 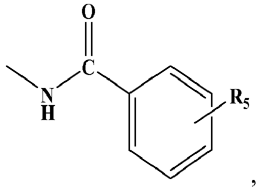 wherein R₅ is H or N₃, 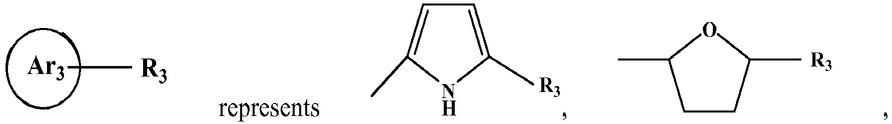 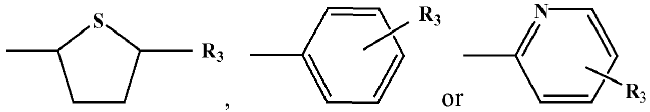 R₃ represents H, C₁₋₃ alkyl, halogen, or -OR, in which R represents H or C₁₋₃ alkyl;
and pharmaceutically acceptable salts thereof, for use in the treatment of breast cancer, wherein said breast cancer is resistant to endocrine therapy.

In a further aspect, the invention relates to a composition comprising
i) a first compound of formula (I) 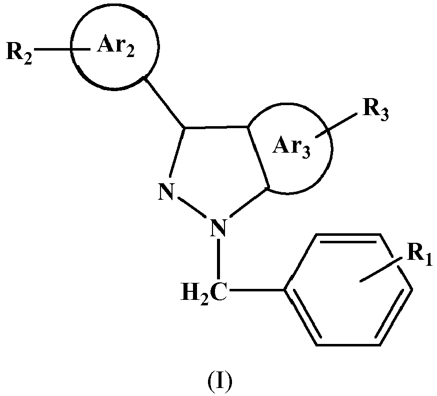 wherein
   R₁ represents H, C₁₋₃ alkyl, halogen, or -OR, in which R represents H or C₁₋₃ alkyl; 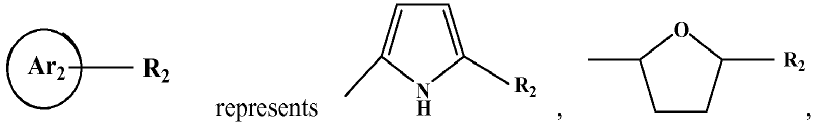 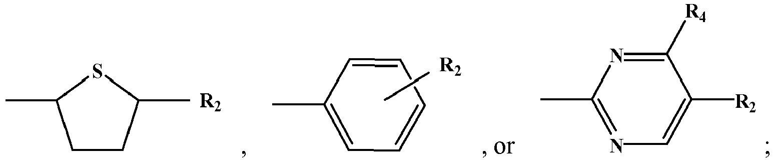 R₂ represents -COOR, -CH₂OR, H, C₁₋₃ alkyl, C₃₋₇ cycloalkyl, or halogen, in which R represents H or C₁₋₃ alkyl;
   R₄ represents H, -NH₂, or 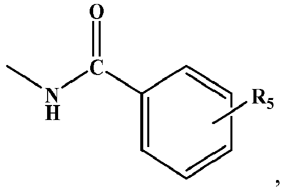 wherein R₅ is H or N₃, 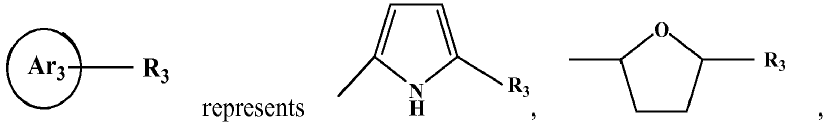 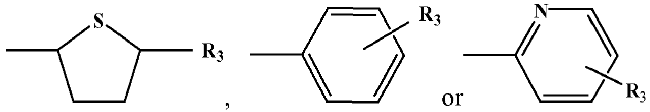 R₃ represents H, C₁₋₃ alkyl, halogen, or -OR, in which R represents H or C₁₋₃ alkyl; or any pharmaceutically acceptable salts thereof, and
i) a second compound selected from the group comprising an estrogen receptor antagonist, a selective estrogen receptor modulator (SERM), an aromatase inhibitor, an ErbB3 inhibitor, an EGFR inhibitor and an mTOR inhibitor.

In a last aspect, the invention relates to the previous composition for use in the treatment of breast cancer, wherein said breast cancer is resistant to endocrine therapy

### DESCRIPTION OF THE FIGURES

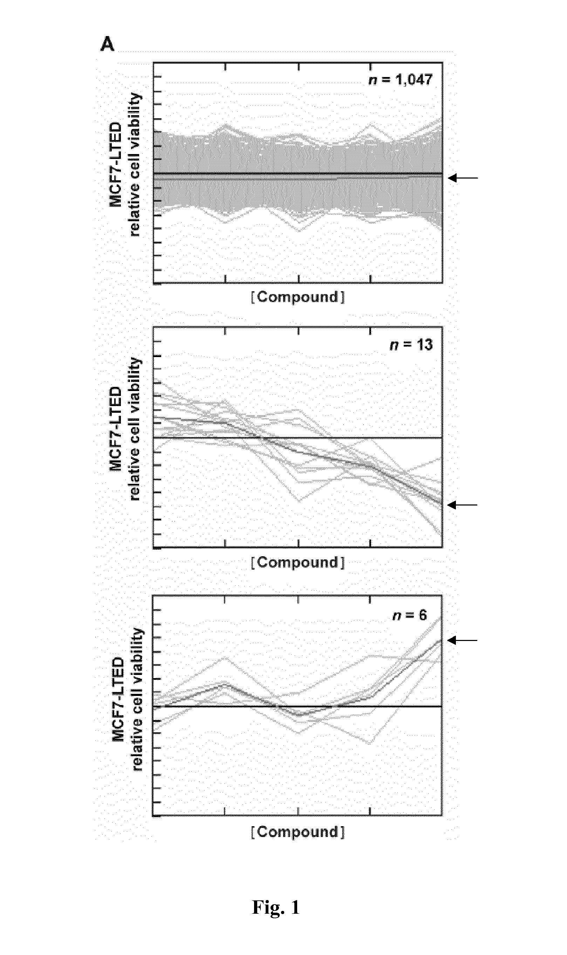

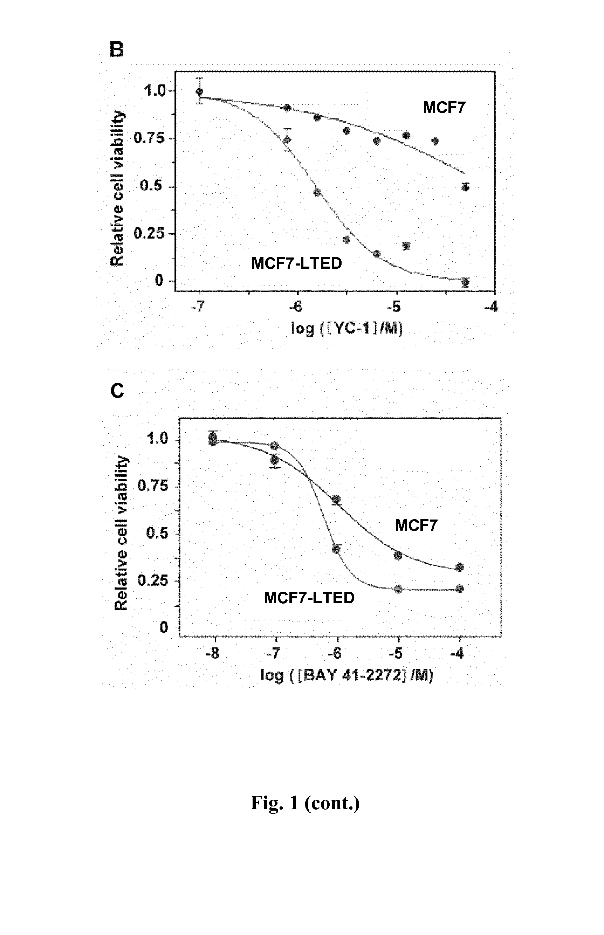

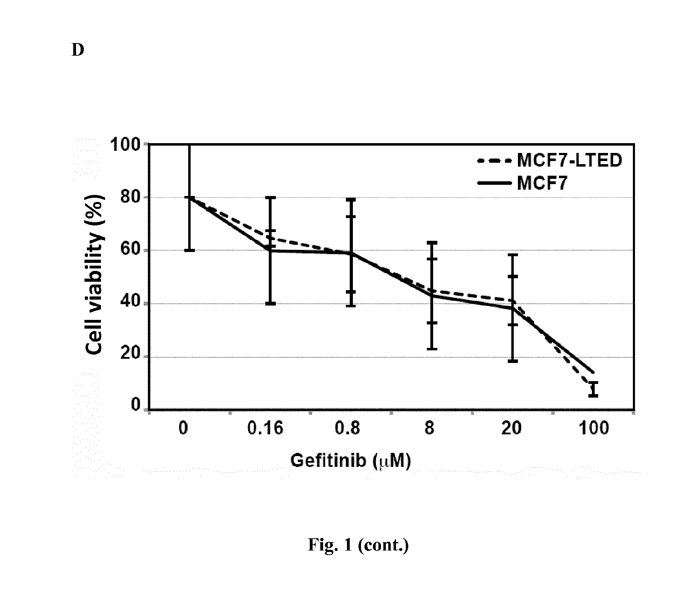

**Figure 1****.** A screening of chemical compounds reducing the viability of MCF7-LTED cells. **(A)** Compounds with no differential effect (top panel), inhibitory effect on MCF7-LTED relative to MCF7 cells (middle panel), and inhibitory effect on MCF7 relative to MCF7-LTED cells (bottom panel). The Y-axis indicates relative viability of MCF7-LTED cells and the X-axis indicates increasing concentrations of the compounds. Wider lines indicated by an arrow correspond to average values. **(B)** Corroboration of the stronger inhibitory effect of YC-1 in MCF7-LTED cells. **(C)** Inhibitory effect of BAY 41-2272. **(D)** Inhibitory effect of gefitinib.

### DETAILED DESCRIPTION OF THE INVENTION

The authors of the present invention have performed a chemical compound screening based on MCF7-LTED (long-term estrogen deprived) cells in order to identify potential therapeutic strategies that could prevent and/or overcome resistance to hormone treatment in breast cancer patients (see Example 1). More than 1,200 compounds were analyzed for their differential effect on cell viability, showing YC-1 as the most active compound in inhibiting cell viability on MCF7-LTED cells (see Figure 1B). YC-1 has also been shown to inhibit cell viability in cell line models of acquired resistance to fulvestrant (an estrogen receptor antagonist) and to the raloxifene analog LY-117018 (a selective estrogen receptor modulator) (see Example 2). Therefore, the authors of the present invention have identified YC-1 as a compound reducing cell viability in cellular models of resistance to endocrine therapies based on aromatase inhibitors, estrogen receptor antagonists and selective estrogen receptor modulators.

### Definitions

The term "aromatase inhibitor" or "AI" refers to a compound that inhibits the enzyme aromatase, which is the enzyme responsible of the conversion of androgens into estrogens by aromatization. Non selective aromatase inhibitors include, without being limited to, sminoglutethimide and testolactone (Teslac). Selective aromatase inhibitors include, without being limited to, anastrazole (Arimidex), letrozole (Femara), exemestane (Aromasin), vorozole (Rivizor), formestane (Lentaron) and fadrozole (Afema). Natural aromatase inhibitors include, without being limited to, resveratrol, nicotine, myosmine, zinc, catechin, chalcones, apigenin, eriodictyol, isoliquiritigenin and mangostin. Other aromatase inhibitors include, without limitation, 4-hydroxyandrostenedione, 1,4,6-androstatrien-3,17-dione (ATD) and 4-androstene-3,6,17-trione (6-OXO). Preferred aromatase inhibitors according to the present invention include, without being limited to, anastrazole, letrozole and exemestane.

The term "BAY 41-2272" (CAS 256376-24-6) relates to a compound structurally related to YC-1 and with formula 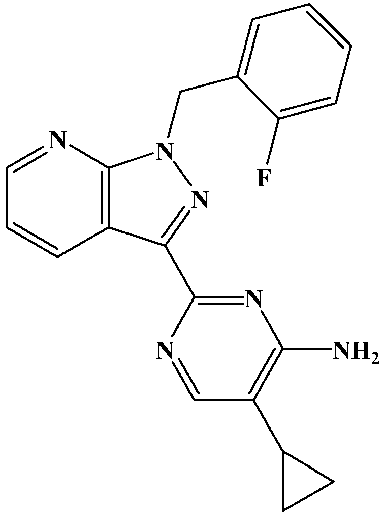 It has been described as an activator of soluble guanylyl cyclase (sGC), acting at a nitric oxide (NO)-independent regulatory site in the sGC α1 subunit. BAY 41-2272 inhibits platelet aggregation (IC₅₀ = 36 nM) and phenylephrine-induced contractions of rabbit aorta (IC₅₀ = 0.30 µM). It has also been described that BAY 41-2272 reduces vascular smooth muscle growth through cAMP- and cGMP-dependent PKA and PKG pathways.

The term "breast cancer" or "breast carcinoma" refers to any malignant proliferative mammary cell disorder, usually occurring in the ducts (the tubes that carry milk to the nipple) and lobules (milk producing glands).

The term "cancer" is referred to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. Biological processes known to be related to cancer include angiogenesis, immune cell infiltration, cell migration and metastasis.

The term "EGFR inhibitor" as used herein relates to therapeutic agents that inhibit, downmodulate, suppress or downregulate EGFR signaling activity. The term is intended to include chemical compounds, such as small molecule inhibitors (e.g., small molecule tyrosine kinase inhibitors) and biologic agents, such as antibodies, interfering RNA (shRNA, siRNA), soluble receptors and the like. EGFR inhibitors according to the invention comprise, without being limited to, antibodies such as cetuximab (Erbitux, a chimeric mouse/human monoclonal antibody), MM-151 (an oligoclonal therapeutic consisting of a mixture of three fully human monoclonal antibodies designed to bind to non-overlapping epitopes of EGFR), Sym004 (a recombinant IgGl antibody product consisting of two antibodies against EGFR), matuzumab (EMD 72000, a humanized monoclonal antibody), panitumumab (a fully human monoclonal antibody specific to EGFR), nimotuzumab (BIOMAb EGFR, TheraCIM, Theraloc, CIMAher, a humanized monoclonal antibody) or mAb 806 (a monoclonal antibody); small molecules inhibiting EGFR signaling such as gefitinib, afatinib, lapatinib, canertinib, erlotinib HC, pelitinib, PKI-166, PD-158780, AG 1478, osrafenib or sunitinib. In a particular embodiment, the EGFR inhibitor according to the invention is an EGFR inhibitor for the treatment of breast cancer.

The term "ErbB3 inhibitor" as used herein, also known as "HER2 inhibitor" or "HER2 receptor inhibitor", relates to therapeutic agents that inhibit, downmodulate, suppress or downregulate activity of ErbB3. The term is intended to include chemical compounds, such as small molecule inhibitors, and biologic agents, such as antibodies, interfering RNA (shRNA, siRNA), soluble receptors and the like. ErbB3 inhibitors include, without limitation, anti-ErbB3 antibodies such as Ab#3, Ab #14, Ab #17, Ab # 19, described in U.S. 7,846,440; antibodies disclosed in US 7,285,649, US20200310557, US20100255010, as well as antibodies IB4C3 and 2D1D12 (U3 Pharma Ag), both of which are described in e.g., US20040197332 and are produced by hybridoma cell lines DSM ACC 2527 or DSM ACC 2517 (deposited at DSMZ); anti-ErbB3 antibody referred to as AMG888 (U3-1287- U3 Pharma Ag and Amgen) described in U.S. patent No. 7,705,130; and monoclonal antibody 8B8 (ATCC® HB-12070™), described in US 5,968,511, and the anti-ErbB3 antibody referred to as AV - 203 (Aveo Pharmaceuticals) which is described in US patent publication No. 20110256154. Other useful anti-ErbB3 antibodies are disclosed in the art in the context of a bispecific antibody (see e.g., B2B3-1 or B2B3-2 in W0/2009126920 and those described in US 7,846,440, US 20090291085. US 20100056761, and US 20100266584. An exemplary anti-ErbB3 monoclonal antibody comprises MM-121, a fully human anti-ErbB3 antibody currently undergoing Phase II clinical trials. Other examples of anti-ErbB3 antibodies include Ab #3, Ab #14, Ab #17 and Ab #19, also described further in WO 20081100624. In a particular embodiment, the ErbB3 inhibitor according to the invention is an ErbB3 inhibitor for the treatment of breast cancer.

The term "endocrine therapy" also known as "hormonal therapy", is defined as a treatment pertaining to blocking or removing hormones. The treatment may remove the gland that synthesizes the hormone or the prohormone, block or inhibit hormone synthesis, prevent or inhibit the hormone from binding to its receptor, or down-regulate or degrade the hormone receptor. In the present invention, an endocrine therapy is related to a therapy in hormone-receptor-positive, particularly estrogen-receptor positive, breast cancer in two possible ways: by lowering the amount of the hormone estrogen level in the body or by blocking the action of estrogen on breast cancer cells. The endocrine therapy according to the present invention comprises a therapy selected from the group comprising an estrogen receptor antagonist, a selective estrogen receptor modulator (SERM) and an aromatase inhibitor. Usually, the endocrine therapy of choice for premenopausal women is based on SERM. Aromatase inhibitors are considered to be first-line endocrine therapy for metastatic estrogen-receptor positive breast cancer in post-menopausal women.

The term "estrogen receptor" or "ER" relates to a member of the nuclear hormone family of intracellular receptors which is activated by estrogen (17-β-estradiol). After activation, it translocates into the nucleus and binds the corresponding target genes. Two different forms of the estrogen receptor, alpha and beta, are known and they are encoded, respectively, by *ESR1* and *ESR2* genes. 17-β-estradiol binds equally well to both alpha and beta receptors, whereas estrone and raloxifene bind preferentially to the alpha receptor and estriol and genistein to the beta receptor. In a particular preferred embodiment of the invention, the estrogen receptor is the estrogen receptor alpha. The term "estrogen receptor alpha" or "ER-alpha" or "ER-α" relates to the alpha form of the estrogen receptor. Natural and synthetic ERα ligands are classified as ERα agonists ((E)2 or 17β estradiol), selective estrogen receptor modulators (SERM) and pure antagonists, according to the response they elicit in hormone-responsive cells. In the context of the invention, ER ligands are those useful in breast cancer treatment and include selective estrogen receptor modulators and ER antagonists.

The term "estrogen receptor antagonist" relates to a compound that inhibits the binding of 17β-estradiol to an estrogen receptor or interferes with the effects of 17β-estradiol binding to an estrogen receptor. Preferred estrogen receptor antagonists according to the present invention are those useful in breast cancer treatment. Estrogen receptor antagonists in breast cancer treatment according to the present invention include fulvestrant (ICI 182,780, Faslodex).

The term "mTOR inhibitor" as used herein relates to therapeutic agents that inhibit, downmodulate, suppress or downregulate mammalian target of rapamycin (mTOR). The term is intended to include chemical compounds, such as small molecule inhibitors (e.g., small molecule serine/threonine kinase inhibitors) and biologic agents, such as antibodies, interfering RNA (shRNA, siRNA), soluble receptors and the like. mTOR inhibitors according to the present invention include, without limitation, rapamycin, sirolimus, temsirolimus, everolimus, ridaforolimus, KU-0063794 (CAS No. 938440-64-3) and WYE-354 (CAS No. 1062169-56-5). In a particular embodiment, the mTOR inhibitor according to the invention is an mTOR inhibitor for the treatment of breast cancer.

The term "resistance" as used in the present invention, relates to the resistance developed to a treatment, in particular to a breast cancer treatment. In general, systemic agents are active at the beginning of therapy in 90% of primary breast cancers and 50% of metastases, which is demonstrated by reduced tumor volume, improved symptoms, and decreased serological tumor markers. However, after a variable period, progression occurs. Multiple mechanisms of both *in vitro* and *in vivo* resistance have been identified, including cellular and biochemical mechanisms (decreased drug accumulation by decreased drug influx, increased drug efflux of altered intracellular drug trafficking; increased inactivation of drug or toxic intermediate; increased repair of or tolerance to drug-induced damage to DNA, protein or membranes; decreased drug activation; altered drug targets; altered co-factor or metabolite levels; altered downstream effectors of cytotoxicity; altered signaling pathway and/or apoptotic responses to drug insult, due to altered gene expression, DNA mutation, amplification, or deletion, altered transcription, posttranscriptional processing, or translation or altered stability of macromolecules) or by *in vivo* mechanisms (pharmacological and anatomic drug barriers; and host-drug interactions such as increased drug inactivation by normal tissues, decreased drug activation by normal tissues or relatively increased normal tissue drug sensitivity). It has been proposed that resistance to therapy is caused in part by a process called genetic amplification. This process allows cancer cells to increase their immortality and invasion properties. Each treatment regimen with a single systemic agent selects a group of cancer cells that is increasingly resistant to therapy, decreasing the rate of response to further therapies.

Resistance to endocrine therapy may develop as *de novo* resistance or as acquired resistance. The term "*de novo* resistance" or "intrinsic resistance" or "primary resistance" relates to resistance present before drug exposure and selection for drug resistance, as a failure to respond to initial drug therapy. The term "acquired resistance" or "secondary resistance" relates to resistance occurring following drug selection, wherein an initial response to drug therapy is followed by subsequence disease progression. In particular, the term "endocrine therapy-resistance" or "hormone resistance" as used herein relates to a subject receiving an endocrine therapy or hormonal therapy and who lacks demonstration of a desired physiological effect, such as a therapeutic benefit, from the administration of the therapy, due to *de novo* endocrine therapy-resistance or to acquired endocrine therapy-resistance. In a particular embodiment, the endocrine therapy-resistance is acquired endocrine therapy-resistance.

The term "selective estrogen receptor modulator" or "SERM" relates to compounds which interfere or inhibit the binding of estrogens to the receptor, regardless of the mechanism. Selective estrogen receptor modulator (SERM) in breast cancer treatment according to the present invention include, without limitation, clomiphene (Clomid, Omifin), DT56a (Femarelle), ormeloxifene or centchroman (Novex-DS, Centron, Sevista), raloxifene (Evista), tamoxifen (tamoxifen citrate; Nolvadex, Istubal, Valodex), toremifene (Acapodene, Fareston), lasofoxifene (Fablyn), afimoxifene (TamoGel), arzoxifene, bazedoxifene (Viviant, Conbriza) and analogues thereof, including the raloxifene analog LY-117018 (CAS No. 63676-25-5).

By "subject" or "individual" or "animal" or "patient" or "mammal," is meant any subject, particularly a mammalian subject, for whom diagnosis, prognosis, or therapy is desired. Mammalian subjects include humans, domestic animals, farm animals, and zoo, sports, or pet animals such as dogs, cats, guinea pigs, rabbits, rats, mice, horses, cattle, cows, and so on. In a preferred embodiment of the invention, the subject is a mammal. In a more preferred embodiment of the invention, the subject is a human.

As used herein, the term "treatment" refers to both therapeutic measures and prophylactic or preventative measures, wherein the object is to prevent or slow down (lessen) an undesired physiological change or disorder, such as the acute rejection after a renal transplant. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The term "YC-1" or "YC 1" as used in the present invention relates to the compound 3-(5'-hydroxymethyl-2'-furyl)-1-benzylindazole (CAS-170632-47-0), also named as [5-(1-benzyl-lh-indazol-3-yl)-2-furyl]methanol, and represented by the following formula

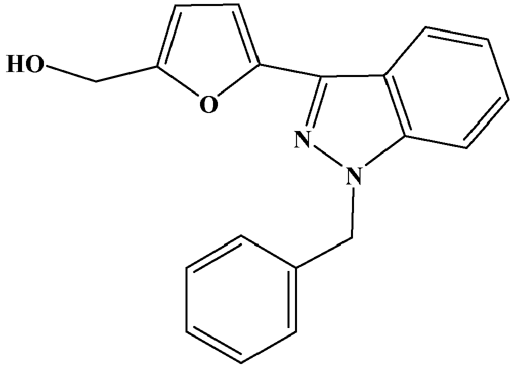

YC-1 was first developed as an agent for treating circulatory diseases after it was proved to inhibit coagulation of platelets and activate soluble guanylyl cyclase (sGC) to inhibit contraction of blood vessels. The synthesis of indole analogs of YC-1 is described in US7176230 B2 and incorporated herein by reference. A list of 1-benzyl-3-(substituted hetaryl)-fused pyrazole derivatives is disclosed in US6180656 Bl. The design and synthesis of photolabile analogues of YC-1 has been described (Hering KW et al. 2006 Bioorganic & Medicinal Chemistry Letters 16:618-621). The synthesis of puropyrazole analogs of YC-1 has been described (Chou LC et al. 2007 Bioorg Med Chem 15(4)1732-1740) as well as other YC-1 analogues (Lee FY et al. 2001 J Med Chem 44(22): 3746-3749).

### Compounds for their use in the treatment of endocrine therapy-resistant breast cancer

The authors of the present invention have found that YC-1 and, to a lesser extent, BAY 41-2272, inhibit cell viability in long-term estrogen-deprived (LTED) MCF7 cells, a cellular model of acquired resistance to aromatase inhibitors in postmenopausal women (Example 1). They have found also that YC-1 inhibits cell viability in cell line models of acquired resistance to fulvestrant, an ER antagonist, and to the raloxifene analog LY-117018, a selective estrogen receptor modulator (see Example 2).

Thus, the invention relates to a compound of formula (I) 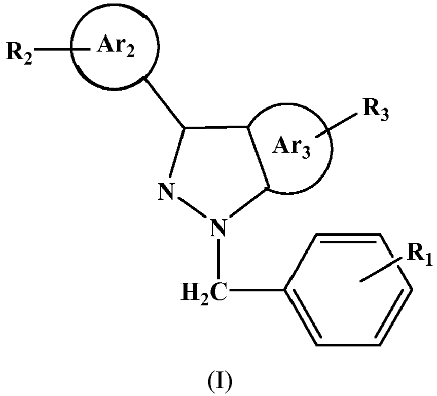 wherein
R₁ represents H, C₁₋₃ alkyl, halogen, or -OR, in which R represents H or C₁₋₃ alkyl; 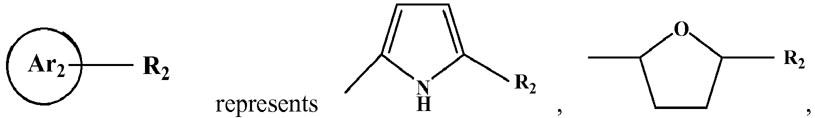 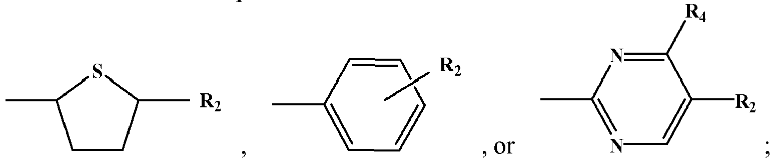 R₂ represents -COOR, -CH₂OR, H, C₁₋₃ alkyl, C₃₋₇ cycloalkyl, or halogen, in which R represents H or C₁₋₃ alkyl;
R₄ represents H, -NH₂, or 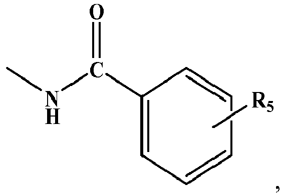 wherein R₅ is H or N₃, 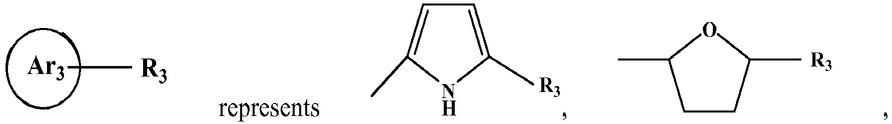 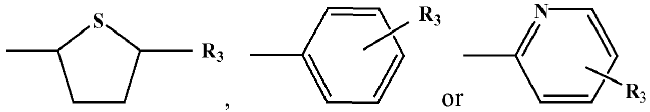 R₃ represents H, C₁₋₃ alkyl, halogen, or -OR, in which R represents H or C₁₋₃ alkyl;
and pharmaceutically acceptable salts thereof, for use in the treatment of breast cancer, wherein said breast cancer is resistant to endocrine therapy.

Alternatively, the invention relates to the use of a compound of formula (I) 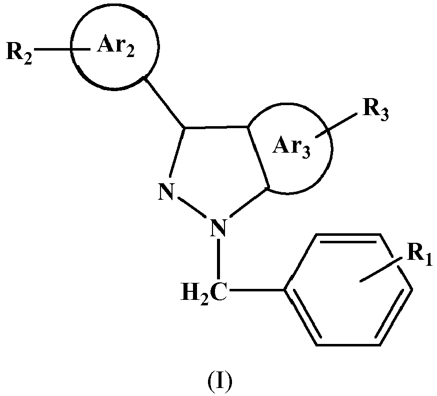 wherein
R₁ represents H, C₁₋₃ alkyl, halogen, or -OR, in which R represents H or C₁₋₃ alkyl; 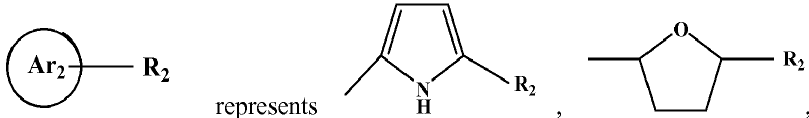 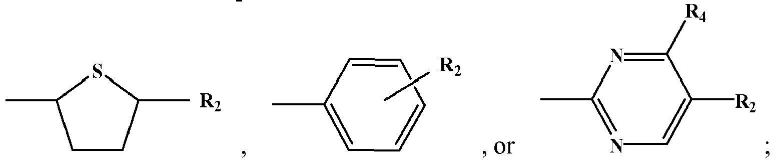 R₂ represents -COOR, -CH₂OR, H, C₁₋₃ alkyl, C₃₋₇ cycloalkyl, or halogen, in which R represents H or C₁₋₃ alkyl;
R₄ represents H, -NH₂, or 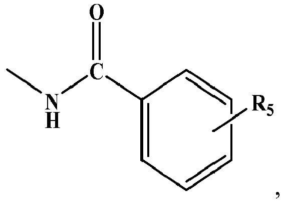 wherein R₅ is H or N₃, 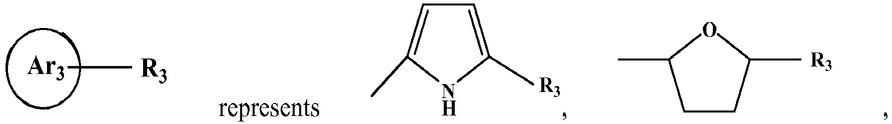 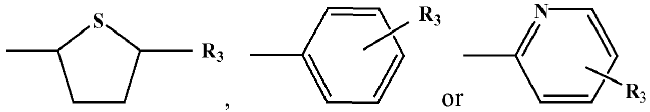 R₃ represents H, C₁₋₃ alkyl, halogen, or -OR, in which R represents H or C₁₋₃ alkyl; and pharmaceutically acceptable salts thereof, in the manufacture of a medicament for the treatment of breast cancer, wherein said breast cancer is resistant to endocrine therapy.

Alternatively, the invention relates to a method for the treatment of breast cancer that involves the administration of a therapeutically effective amount of a compound of formula (I) 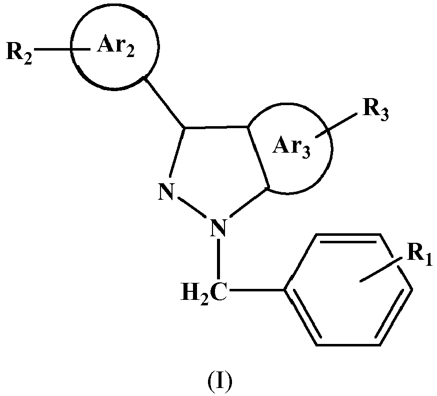 wherein
R₁ represents H, C₁₋₃ alkyl, halogen, or -OR, in which R represents H or C₁₋₃ alkyl; 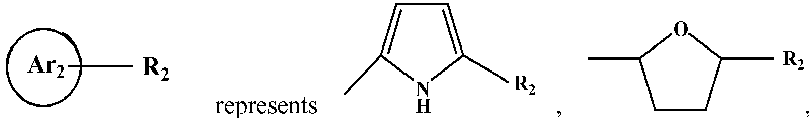 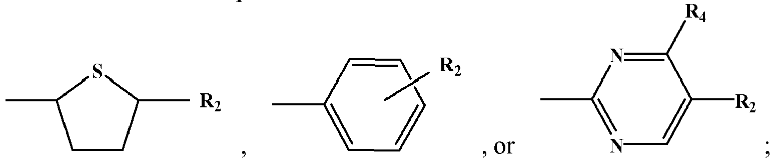 R₂ represents -COOR, -CH₂OR, H, C₁₋₃ alkyl, C₃₋₇ cycloalkyl, or halogen, in which R represents H or C₁₋₃ alkyl;
R₄ represents H, -NH₂, or 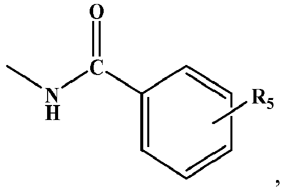 wherein R₅ is H or N₃, 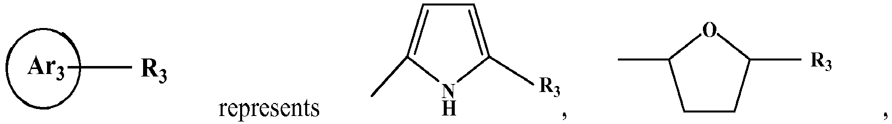 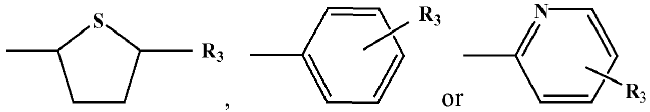 R₃ represents H, C₁₋₃ alkyl, halogen, or -OR, in which R represents H or C₁₋₃ alkyl; and pharmaceutically acceptable salts thereof; to a subject suffering from breast cancer, wherein said breast cancer is resistant to endocrine therapy.

The compound according to the invention for use in the treatment of breast cancer is a 1-benzyl-3-(substituted aryl) condensed pyrazol of the following general formula (I): 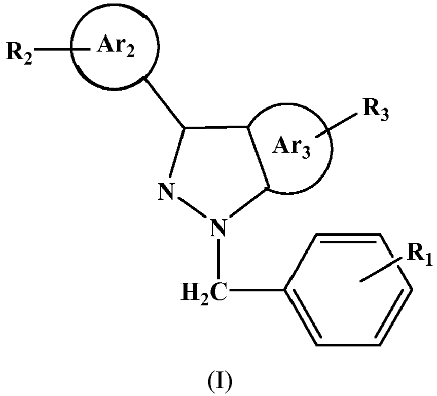 wherein
R₁ represents H, C₁₋₃ alkyl, halogen, or -OR, in which R represents H or C₁₋₃ alkyl; 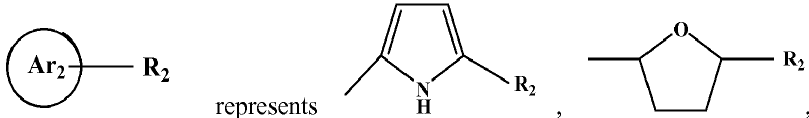 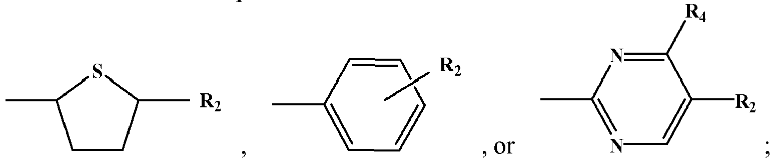 R₂ represents -COOR, -CH₂OR, H, C₁₋₃ alkyl, C₃₋₇ cycloalkyl, or halogen, in which R represents H or C₁₋₃ alkyl;
R₄ represents H, -NH₂, or 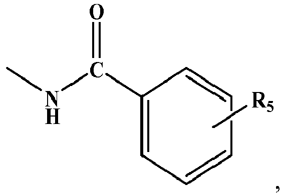 wherein R₅ is H or N₃, 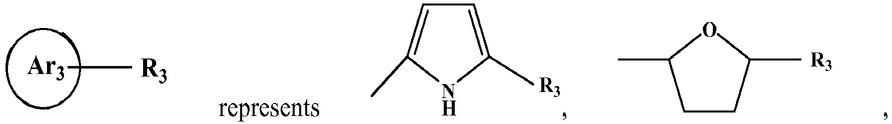 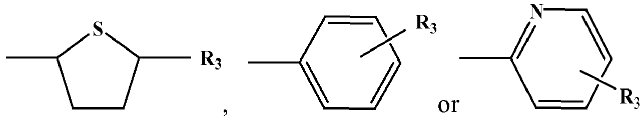 and R₃ represents H, C₁₋₃ alkyl, halogen, or -OR, in which R represents H or C₁₋₃ alkyl; as well as pharmaceutically acceptable salts thereof, for use in the treatment of breast cancer, wherein said breast cancer is resistant to endocrine therapy.

C₁₋₃ alkyl radicals may be straight-chain or branched. This also applies when they are substituted or when they are part of other groups. Examples of C₁₋₃ alkyl groups are methyl, ethyl, n-propyl and isopropyl. The term C₁₋₃ alkyl here also includes unsaturated alkyl radicals, in particular alkyl radicals which contain one or two double bonds or one triple bond.

A cycloalkyl radical is a radical derived from cycloalkane containing from 3 to 7 (C₃₋₇ cycloalkyl), preferably from 3 to 6 (C₃₋₆ cycloalkyl) carbon atoms. Illustrative examples of cycloalkyl groups include cyclopropyl, cyclobutyl, cyclopentyl, cyclohexyl, etc.

If they are appropriately substituted, the compounds of the formula I may be present in stereoisomeric forms. If the compounds of the formula I contain one or more centers of symmetry, these are identical or different from each other and have the S configuration or the R configuration. The invention includes all possible stereoisomers, for example enantiomers and diastereomers, and mixtures of two or more so stereoisomeric forms, for example mixtures of enantiomers and/or diastereomers, in all ratios. Thus, the invention provides enantiomers in enantiomerically pure form, both as levorotatory and as dextrorotatory antipodes, in the form of racemates and in the form of mixtures of the two enantiomers in all ratios. In the case of a cis/trans isomerism, the invention provides both the cis form and the trans form and mixtures of these forms. The preparation of individual stereoisomers can be carried out, if desired, by separation of a mixture by customary methods, for example by chromatography or crystallization, by the use of stereochemically uniform starting materials for the synthesis or by stereoselective synthesis. The separation of a mixture of stereoisomers can be carried out at the stage of the compounds of the formula I or during the synthesis.

If mobile hydrogen atoms are present, the present invention also includes all tautomeric forms of the compounds of the formula I, for example lactam/lactim tautomers.

If the compounds of the formula I contain one or more acidic or basic groups, the invention also provides the corresponding physiologically or toxicologically tolerable salts, in particular the pharmaceutically acceptable salts. Thus, the compounds of the formula I which contain one or more acidic groups, for example COOH groups or N-acylsulfonamido groups, may be present on these groups and may be used according to the invention, for example as alkali metal salts, alkaline earth metal salts or as ammonium salts. Examples of such salts are sodium salts, potassium salts, calcium salts, magnesium salts or salts with ammonia or organic amines, such as, for example, ethylamine, ethanolamine, triethanolamine or amino acids. Compounds of the formula I which contain one or more basic groups, i.e., groups which can be protonated, can be present and can be used according to the invention in the form of their acid addition salts with inorganic or organic acids which, for example as salts with hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfarninic acid, phenylpropionic acid, gluconic acid, ascorbic acid, isonicotinic acid, citric acid, adipic acid, etc.

If the compounds of the formula I simultaneously contain acidic and basic groups in the molecule, the invention also includes, in addition to the salt forms mentioned, inner salts, so-called betaines. Salts can be obtained from the compounds of the formula I by customary methods which are known to the person skilled in the art, for example by combination with an organic or inorganic acid or base in a solvent or dispersant, or else by anion exchange or cation exchange from other salts. The present invention also includes all salts of the compounds of the formula I which, owing to low physiological compatibility, are not directly suitable for use in pharmaceuticals but which can be used, for example, as intermediates for chemical reactions or for the preparation of physiologically acceptable salts.

The present invention furthermore includes all solvates of compounds of the formula I, for example hydrates or adducts with alcohols, and also derivatives of the compounds of the formula I, for example esters, prodrugs and metabolites, which act like the compounds of the formula I.

In a particular embodiment of the invention, the compound for use in the treatment of endocrine therapy-resistant breast cancer has the following formula (I) 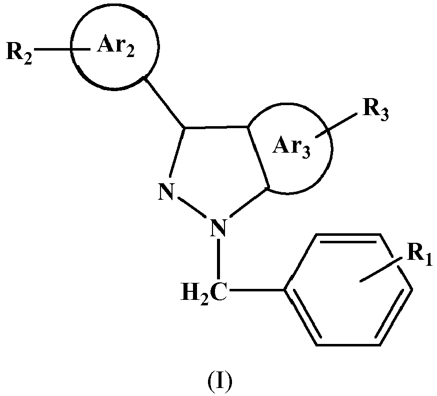 wherein
R₁ represents H, C₁₋₃ alkyl, halogen, or -OR, in which R represents H or C₁₋₃ alkyl; 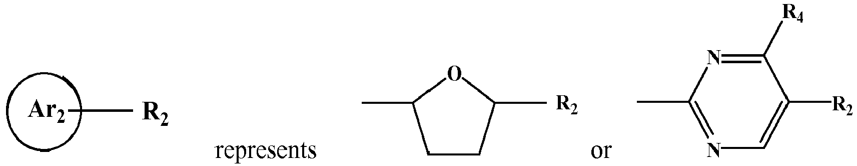 R₂ represents -COOR, -CH₂OR, H, C₁₋₃ alkyl, C₃₋₇ cycloalkyl, or halogen, in which R represents H or C₁₋₃ alkyl;
R₄ represents H, -NH₂, or 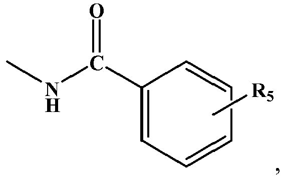 wherein R₅ is H or N₃, 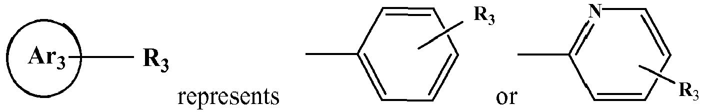 R₃ represents H, C₁₋₃ alkyl, halogen, or -OR, in which R represents H or C₁₋₃ alkyl; and pharmaceutically acceptable salts thereof.

In a more particular embodiment of the invention, the compound for use in the treatment of endocrine therapy-resistant breast cancer has the following formula (I) 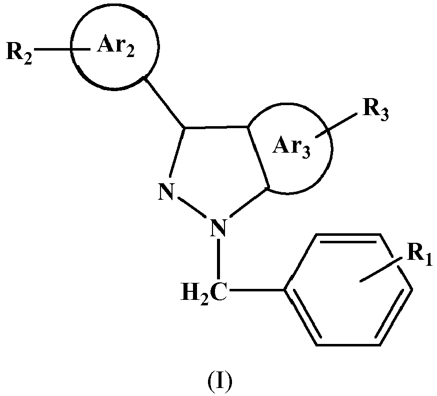 wherein
R₁ represents H, C₁₋₃ alkyl or halogen; 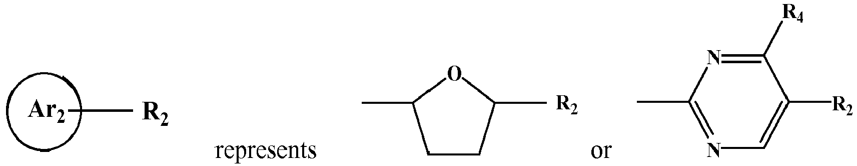 R₂ represents -COOR, -CH₂OR, H, C₁₋₃ alkyl, C₃₋₇ cycloalkyl, or halogen, in which R represents H or C₁₋₃ alkyl;
R₄ represents H, -NH₂, or 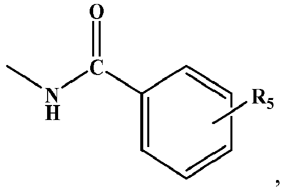 wherein R₅ is H or N₃, 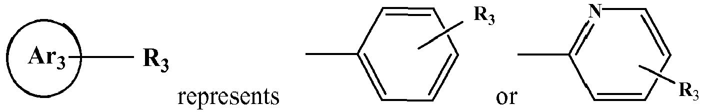 R₃ represents H, C₁₋₃ alkyl or halogen;
and pharmaceutically acceptable salts thereof.

In a particular preferred embodiment of the invention, the compound of formula (I) is is a compound having the formula 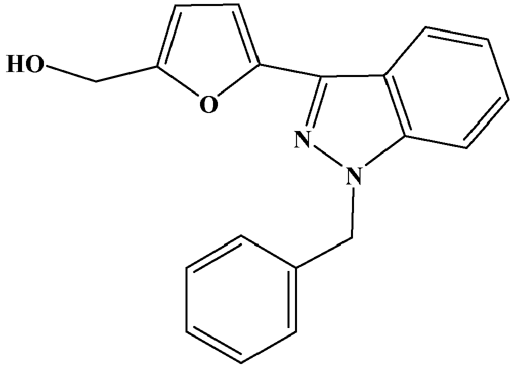 which is also referred herein as YC-1.

Preparation methods for compounds of formula (I), as well as a number of indazole analogues of YC-1, are disclosed in EP0667345-Al and incorporated herein by reference.

The present invention also relates to compounds structurally related to YC-1 for their use in the treatment of breast cancer, particularly endocrine therapy-resistant breast cancer. Compounds structurally related to YC-1 include pyrazolopyridine BAY 41-2272 (CAS 256376-24-6) according to the following formula,

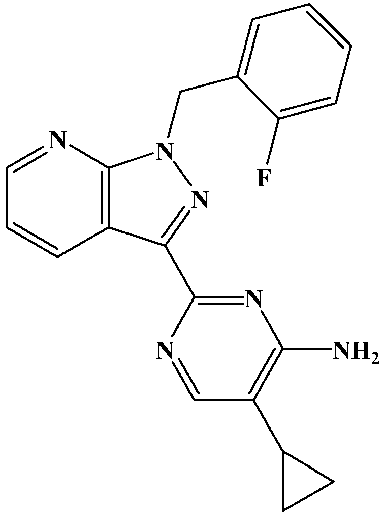

Thus, in a preferred embodiment, the invention relates to a compound selected from the group consisting of a compound having the formula 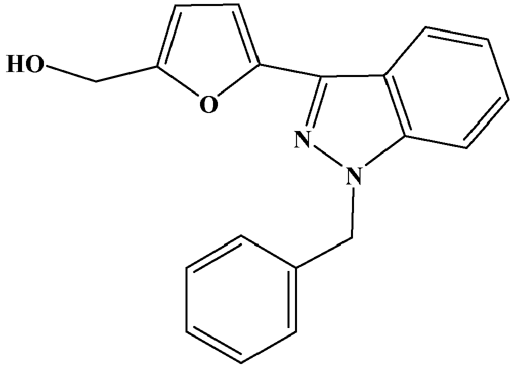 and a compound having the formula 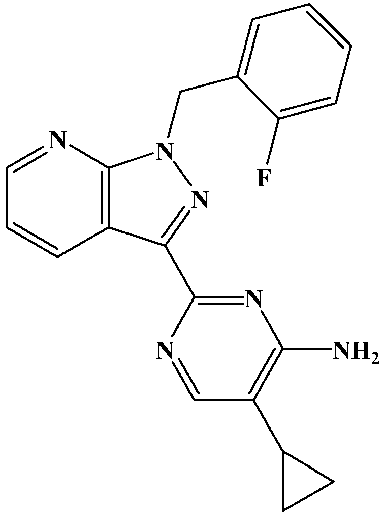 and pharmaceutically acceptable salts thereof, for use in the treatment of breast cancer, wherein said breast cancer is resistant to endocrine therapy.

Compounds for their use according to the present invention also include the BAY 41-2272 derivatives ortho-(BAY 50-6038), meta-(BAY 51-9491) and para-PAL-(BAY 50-8364).

BAY 41-2272 derivative ortho-(BAY 50-6038) has the following formula:

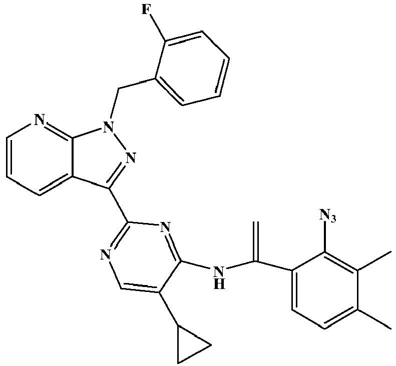

BAY 41-2272 derivative meta-(BAY 51-9491) has the following formula:

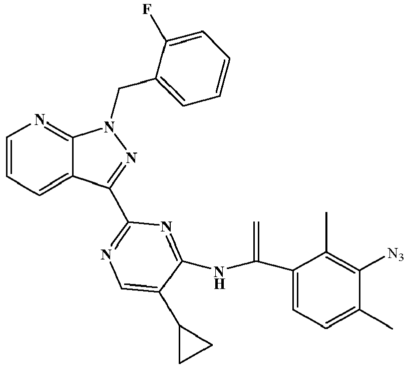

BAY 41-2272 derivative para-PAL-(BAY 50-8364) has the following formula:

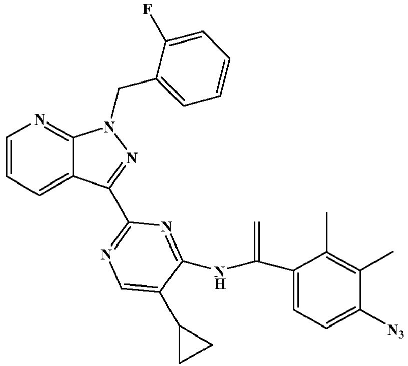

As the skilled person acknowledges, effectiveness of a breast cancer therapy, in particular an endocrine therapy, is demonstrated by reduced tumor volume, improved symptoms, and/or decreased serological tumor markers. However, after a variable period, progression due to therapy resistance may occur. Tumor resistance, in particular breast tumor resistance, to an endocrine therapy can develop as *de novo* resistance, wherein the tumor is not responsive to the therapy, or as acquired resistance, wherein, after initial response to the therapy, the tumor develops resistance leading to disease progression.

In the context of the invention, the compounds as described previously are used in the treatment of breast cancer, wherein the breast cancer is resistant to endocrine therapy. In a particular embodiment, the compounds as described previously are used in the treatment of breast cancer, wherein the breast cancer is resistant to endocrine therapy by acquired resistance to said endocrine therapy.

In a particular embodiment, the endocrine therapy is selected from the group comprising an estrogen receptor antagonist, a selective estrogen receptor modulator (SERM) and an aromatase inhibitor.

In a preferred embodiment, the estrogen receptor antagonist is fulvestrant.

In a preferred embodiment, the selective estrogen receptor modulator is selected from the group comprising clomiphene, DT56a, ormeloxifene, raloxifene, tamoxifen, toremifene, lasofoxifene, afimoxifene, arzoxifene and bazedoxifene. In a more preferred embodiment, the selective estrogen receptor modulator is tamoxifen.

In a preferred embodiment, the aromatase inhibitor is selected from the group comprising sminoglutethimide, testolactone, anastrazole, letrozole, exemestane, vorozole, formestane, fadrozole, resveratrol, nicotine, myosmine, zinc, catechin, chalcones, apigenin, eriodictyol, isoliquiritigenin, mangostin, 4-hydroxyandrostenedione, 1,4,6-androstatrien-3,17-dione (ATD) and 4-androstene-3,6,17-trione (6-OXO). In a more preferred embodiment, the aromatase inhibitor is selected from the group comprising anastrazole, letrozole and exemestane.

A breast cancer can be identified as being resistant to an endocrine therapy using methods available to the person skilled in the art. Regarding *de novo* resistance, lack of expression of ERα is considered the primary mechanism of *de novo* resistance to tamoxifen. A second marker of tamoxifen resistance is CYP2D6, since patients carrying inactive alleles of cytochrome P450 2D6 (CYP2D6) fail to convert tamoxifen to its active metabolite, endoxifen, and are consequently less responsive to tamoxifen. Regarding acquired resistance to endocrine therapy, deregulation of various aspects of estrogen signaling is a common mechanism for resistance, but unrelated mechanisms that provide tumor cells with alternative proliferative and survival stimuli also confer resistance. Pathways associated with tamoxifen resistance include ER signaling, growth factor receptors and cytoplasmic signaling (MAPK signaling, PI3K signaling, SRC), cell cycle (cyclins D1 and E1; MYC; CDK inhibitors p21 and p27; RB) and apoptosis and cell survival signaling (BCL-2, BIK, BAD, IRF1, XBP1) (Musgrove EA & Sutherland RL 2009 Nat Rev 9: 631-643). Amplification or overexpression of *ERBB2* (human epidermal growth factor receptor 2 [HER2]) proto-oncogene is linked with endocrine resistance. Hyperactivation of the phosphatidylinositol 3-kinase (PI3K) pathway, the most frequently mutated pathway in breast cancer, promotes antiestrogen resistance (Miller TM et al. 2011 Amer Soc Clin Oncol 29(33): 4452-4461). Multiple mechanisms have been described whereby breast cancers appear unresponsive to endocrine therapy including, without limitation, tumor insensitivity to hormone therapy, activation of hormone signaling pathways by non-endocrine pathways, ineffective or compromised therapy (inherent lack of potency, compensatory mechanism or poor pharmacokinetics), and domination of cell survival.

Various techniques have been developed in the art for monitoring tumor response to a therapy, wherein measuring tumor size and/or shrinkage on computed thomography (CT) or other anatomic imaging modalities represents a current standard. Imaging of tumor metabolism with PET and the glucose analog 18F-FDG is contemplated as well for assessing the effects of therapy objectively and quantitatively. Due to relatively easy access to breast tumors, biopsies may be taken sequentially before and during treatment and analyzed to monitor the effects of treatment on molecular processes, including development of tumor resistance. Methods for obtaining a biopsy sample, particularly a breast tumor biopsy sample, are known by the skilled person and include surgical resection of a tissue mass or microdissection or other known cell separation methods, the cells can additionally be obtained by aspiration cytology by means of the pricking with a thin needle connected to a syringe. Monitoring of the response to an endocrine therapy and the development of resistance can be performed by analyzing the gene expression of ER as well as the expression of estrogen-regulated genes including, without limitation, KIAA0101, SERPINA3, IRS1, TFF3 and TFF1 as markers of (anti)estrogenic responses, and also Ki67 and expression of cell cycle/DNA synthesis genes including, without limitation, CDC, CKS2, Cyclin B1, TYMS and PCNA as markers of proliferation. Efficiency of a treatment, particularly an endocrine treatment for breast cancer, may be monitored by, without limitation, measuring estrogen levels (particularly in the case of aromatase inhibitors), downstream signaling of ER (particularly in the case of selective ER modulators and selective ER down-regulators).

Methods for determining estrogen levels in a sample of a subject are known in the art and include, without limitation, competitive immunoassays (e.g., by DPC IMMULITE 2000 Analyzer, NY; DPC IMMULITE Analyzer, Ireland), as well as those described in EP0817970A1.

Methods for determining the gene expression in sample of a subject can be determined by measuring the levels of mRNA encoded by said gene, or by measuring the levels of the protein encoded by said gene, which are known by the skilled person and include, without limitation, immunohistochemistry, time resolved fluorescence immunoassays (TR-FIA), radioimmunoassays, enzyme immunoassays (e.g., ELISA), immunofluorescence immunoprecipitation, latex agglutination, hemagglutination, which are conventional methods well-known in the art for protein expression analysis, as well as qPCR, northern blot, RNA dot blot, TaqMan, tag based methods such as serial analysis of gene expression (SAGE) including variants such as LongSAGE and SuperSAGE, microarrays, Fluorescence In Situ Hybridization, including variants such as Flow-FISH, qFiSH and double fusion FISH (D-FISH), which are conventional methods well-known in the art for mRNA expression analysis.

Appropriate amounts of compounds according to the present invention can be formulated with pharmaceutically acceptable excipients and/or carriers to obtain a pharmaceutical composition for use in the treatment of breast cancer. A composition that includes a compound according to the invention can be delivered to a subject by a variety of routes including, without limitation, systemically delivery, e.g., by intravenous, subcutaneous or intramuscular injection. Additionally, it is also possible to administer the composition of the invention intranasally which allows systemic administration by a non-aggressive mode of administration. Also, intraventricular administration may be adequate. A preferred route of delivery is intravenous injection.

Those skilled in the art are familiar with the principles and procedures discussed in widely known and available sources as Remington's Pharmaceutical Science (17th Ed., Mack Publishing Co., Easton, Pa., 1985) and Goodman and Gilman's The Pharmaceutical Basis of Therapeutics (8th Ed., Pergamon Press, Elmsford, N.Y., 1990) both of which are incorporated herein by reference.

In a preferred embodiment of the present invention, the compounds of the invention are formulated in accordance with standard procedure as a pharmaceutical composition adapted for delivered administration to human beings and other mammals. Typically, compositions for intravenous or intraventricular administration are solutions in sterile isotonic aqueous buffer.

Where necessary, the compound of the invention is comprised in a composition also including a solubilizing agent and a local anesthetic to ameliorate any pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where the composition is to be administered by infusion, it can be dispensed with an infusion bottle containing sterile pharmaceutical grade water or saline. Where the composition is administered by injection, an ampule of sterile water for injection or saline can be provided so that the ingredients may be mixed prior to administration.

In cases other than intravenous administration, the composition can contain minor amounts of wetting or emulsifying agents, or pH buffering agents. The composition can be a liquid solution, suspension, emulsion, gel, polymer, or sustained release formulation. The composition can be formulated with traditional binders and carriers, as would be known in the art. Formulations can include standard carriers such as pharmaceutical grades of mannitol, lactose, starch, magnesium stearate, sodium saccharide, cellulose, magnesium carbonate, etc., inert carriers having well established functionality in the manufacture of pharmaceuticals. Various delivery systems are known and can be used to administer a therapeutic of the present invention including encapsulation in liposomes, microparticles, microcapsules and the like.

In yet another preferred embodiment, therapeutics containing the compounds of the invention can be formulated as neutral or salt forms. Pharmaceutically acceptable salts include those formed with free amino groups such as those derived from hydrochloric, phosphoric, acetic, oxalic, tartaric acids and the like, and those formed with free carboxyl groups such as those derived from sodium, potassium, ammonium, calcium, ferric hydroxides, isopropylamine, thriethylamine, 2-ethylamino ethanol, histidine, procaine or similar.

The pharmaceutical compositions containing the compounds according to the invention can occur at any pharmaceutical form of administration considered appropriate for the selected administration route, for example, by systemic, oral, parenteral or topical administration, for which it will include the pharmaceutically acceptable excipients necessary for formulation of the desired method of administration.

The effective quantity of the compounds of the invention can vary within a wide range and, in general, will vary depending on the particular circumstances of application, duration of the exposure and other considerations.

Solid dosage forms for oral administration may include conventional capsules, sustained release capsules, conventional tablets, sustained-release tablets, chewable tablets, sublingual tablets, effervescent tablets, pills, suspensions, powders, granules and gels. At these solid dosage forms, the active compounds can be mixed with at least one inert excipient such as sucrose, lactose or starch. Such dosage forms can also comprise, as in normal practice, additional substances other than inert diluents, e.g. lubricating agents such as magnesium stearate. In the case of capsules, tablets, effervescent tablets and pills, the dosage forms may also comprise buffering agents. Tablets and pills can be prepared with enteric coatings.

Liquid dosage forms for oral administration may include emulsions, solutions, suspensions, syrups and elixirs pharmaceutically acceptable containing inert diluents commonly used in the technique, such as water. Those compositions may also comprise adjuvants such as wetting agents, emulsifying and suspending agents, and sweetening agents, flavoring and perfuming agents.

Injectable preparations, for example, aqueous or oleaginous suspensions, sterile injectable may be formulated according with the technique known using suitable dispersing agents, wetting agents and/or suspending agents. Among the acceptable vehicles and solvents that can be used are water, Ringer's solution and isotonic sodium chloride solution. Sterile oils are also conventionally used as solvents or suspending media.

For topical administration, compounds of the invention can be formulated as creams, gels, lotions, liquids, pomades, spray solutions, dispersions, solid bars, emulsions, microemulsions and similars which may be formulated according to conventional methods that use suitable excipients, such as, for example, emulsifiers, surfactants, thickening agents, coloring agents and combinations of two or more thereof.

Additionally, the compounds of the invention may be administered in the form of transdermal patches or iontophoresis devices. In one embodiment, the compounds of the invention are administered as a transdermal patch, for example, in the form of sustained-release transdermal patch. Suitable transdermal patches are described in more detail in, for example, US5262165, US5948433, US6010715 and US6071531.

The compositions comprising the compounds of the invention can additionally include conventional excipients, ie pharmaceutically acceptable carriers suitable for parenteral application which do not react damaging with the active compounds. Suitable pharmaceutically acceptable vehicles include, for example, water, salt solutions, alcohol, vegetable oils, polyethylene glycols, gelatin, lactose, amylose, magnesium stearate, talc, surfactants, silicic acid, viscous paraffin, perfume oil, monoglycerides and diglycerides of fatty acids, fatty acid esters petroetrals, hydroxymethyl cellulose, polyvinylpyrrolidone and similars.

Several drug delivery systems are known and can be used to administer the compounds or compositions of the invention, including, for example, encapsulation in liposomes, microbubbles, emulsions, microparticles, microcapsules and similars. The required dosage can be administered as a single unit or in a sustained release form.

Sustainable-release forms and appropriate materials and methods for their preparation are described in, for example, "Modified-Release Drug Delivery Technology", Rathbone, M. J. Hadgraft, J. and Roberts, M. S. (eds.), Marcel Dekker, Inc., New York (2002), "Handbook of Pharmaceutical Controlled Release Technology", Wise, D. L. (ed.), Marcel Dekker, Inc. New York, (2000). In one embodiment of the invention, the orally administrable form of a compound according to the invention is in a sustained release form further comprises at least one coating or matrix. The coating or sustained release matrix include, without limitation, natural polymers, semisynthetic or synthetic water-insoluble, modified, waxes, fats, fatty alcohols, fatty acids, natural semisynthetic or synthetic plasticizers, or a combination of two or more of the them.

Enteric coatings may be applied using conventional processes known to experts in the art, as described in, for example, Johnson, J. L., "Pharmaceutical tablet coating", Coatings Technology Handbook (Second Edition), Satas, D. and Tracton, A. A. (eds), Marcel Dekker, Inc. New York, (2001), Carstensen, T., "Coating Tablets in Advanced Pharmaceutical Solids", Swarbrick, J. (ed.), Marcel Dekker, Inc. New York (2001), 455-468.

Even though individual needs vary, determination of optimal ranges for effective amounts of the compound of the invention belongs to the common experience of those experts in the art. In general, the dosage needed to provide an effective amount of such compound, which can be adjusted by one expert in the art will vary depending on age, health, fitness, sex, diet, weight, degree of alteration of the receptor, frequency of treatment and the nature and extent of impairment or illness, medical condition of the patient, route of administration, pharmacological considerations such as activity, efficacy, pharmacokinetic and toxicology profile of the particular compound used, if using a system drug delivery, and if the compound is administered as part of a combination of drugs.

The amount of the compound according to the invention that is effective in the treatment of a particular disorder or condition will depend on the nature of the disorder or condition, in particular breast cancer, and can be determined by conventional clinical techniques, including reference to Goodman and Gilman, supra; The Physician's Desk Reference, Medical Economics Company, Inc., Oradell, NJ, 1995, and Drug Facts and Comparisons, Inc., St. Louis, MO, 1993. The precise dose to use in the formulation will also depend on the route of administration, and severity of the disease or disorder, and should be decided in the physician's opinion and the patient's circumstances.

### Compositions of the invention

In a further aspect, the present invention relates to a composition comprising
i) a first compound of formula (I) 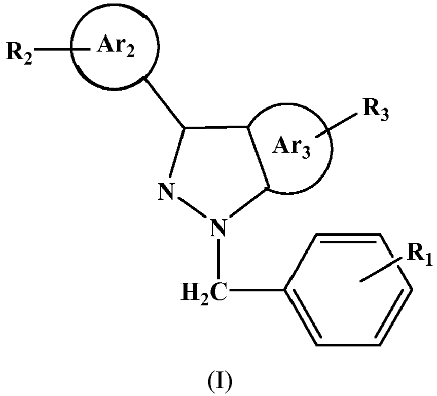 wherein
   R₁ represents H, C₁₋₃ alkyl, halogen, or -OR, in which R represents H or C₁₋₃ alkyl; 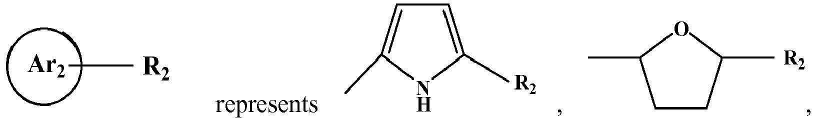 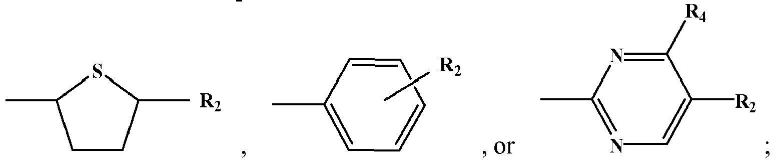 R₂ represents -COOR, -CH₂OR, H, C₁₋₃ alkyl, C₃₋₇ cycloalkyl, or halogen, in which R represents H or C₁₋₃ alkyl;
   R₄ represents H, -NH₂, or 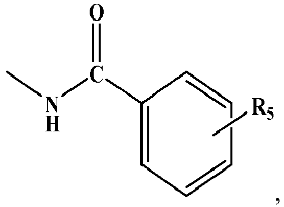 wherein R₅ is H or N₃, 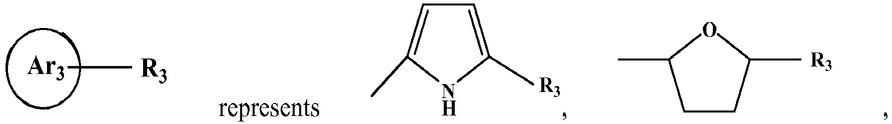 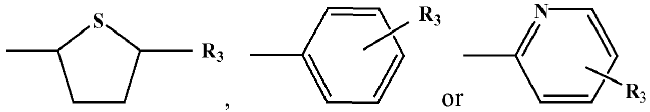 R₃ represents H, C₁₋₃ alkyl, halogen, or -OR, in which R represents H or C₁₋₃ alkyl; or any pharmaceutically acceptable salts thereof, and
ii) a second compound selected from the group comprising an estrogen receptor antagonist, a selective estrogen receptor modulator (SERM), an aromatase inhibitor, an ErbB3 inhibitor, an EGFR inhibitor and an mTOR inhibitor.

In a particular embodiment of the composition according to the invention, the first compound is a compound of formula (I) as above wherein
R₁ represents H, C₁₋₃ alkyl, halogen, or -OR, in which R represents H or C₁₋₃ alkyl; 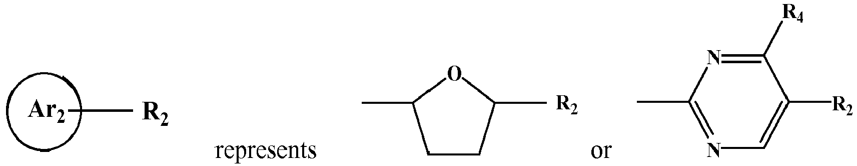 R₂ represents -COOR, -CH₂OR, H, C₁₋₃ alkyl, C₃₋₇ cycloalkyl, or halogen, in which R represents H or C₁₋₃ alkyl;
R₄ represents H, -NH₂, or 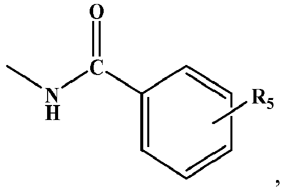 wherein R₅ is H or N₃, 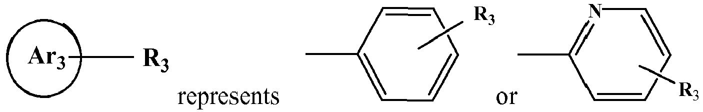 and R₃ represents H, C₁₋₃ alkyl, halogen, or -OR, in which R represents H or C₁₋₃ alkyl; or any pharmaceutically acceptable salt thereof.

In a more particular embodiment of the composition according to the invention, the first compound is a compound of formula (I) as above wherein
R₁ represents H, C₁₋₃ alkyl or halogen; 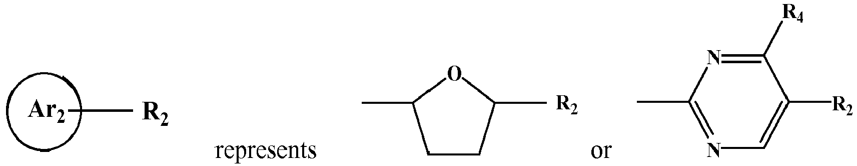 R₂ represents -COOR, -CH₂OR, H, C₁₋₃ alkyl, C₃₋₇ cycloalkyl, or halogen, in which R represents H or C₁₋₃ alkyl;
R₄ represents H, -NH₂, or 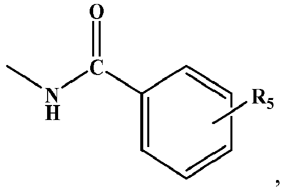 wherein R₅ is H or N₃, 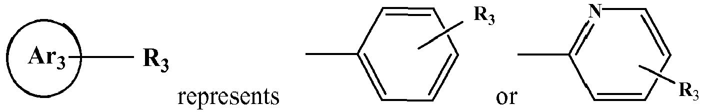 R₃ represents H, C₁₋₃ alkyl or halogen; or any pharmaceutically acceptable salt thereof.

In a preferred embodiment of the composition according to the invention, the first compound is selected from the compound having the formula 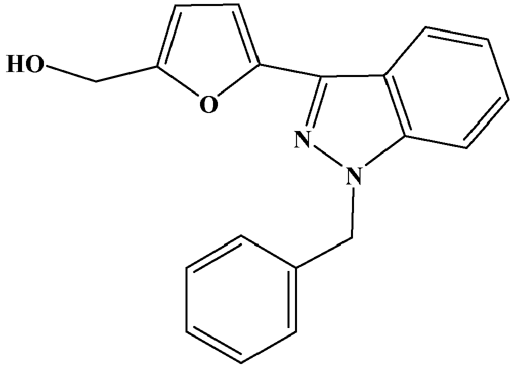 and a compound having the formula

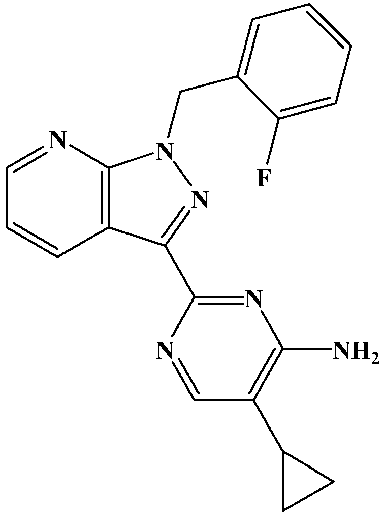

As previously mentioned, the composition of the invention comprises a first compound of formula (I) as previously described and second compound, wherein the second compound is selected from the group comprising an estrogen receptor antagonist, a selective estrogen receptor modulator (SERM), an aromatase inhibitor, an ErbB3 inhibitor, an EGFR inhibitor and an mTOR inhibitor.

Estrogen receptor antagonists, selective estrogen receptor modulators (SERM), aromatase inhibitors, ErbB3 inhibitors, EGFR inhibitors and mTOR inhibitors according to the invention have been described above. In the context of the present invention, preferred estrogen receptor antagonists, selective estrogen receptor modulators (SERM), aromatase inhibitors, ErbB3 inhibitors, EGFR inhibitors and mTOR inhibitors are those used in the treatment of cancer, particularly in the treatment of breast cancer, more particularly in the treatment of breast cancer resistant to endocrine therapy.

In a particular embodiment of the composition of the invention, the estrogen receptor antagonist is fulvestrant.

In a particular embodiment of the composition of the invention, the selective estrogen receptor modulator (SERM) is selected from the group comprising clomiphene, DT56a, ormeloxifene, raloxifene, tamoxifen, toremifene, lasofoxifene, afimoxifene, arzoxifene and bazedoxifene.

In a particular embodiment of the composition of the invention, the aromatase inhibitor is selected from the group comprising sminoglutethimide, testolactone, anastrazole, letrozole, exemestane, vorozole, formestane, fadrozole, resveratrol, nicotine, myosmine, zinc, catechin, chalcones, apigenin, eriodictyol, isoliquiritigenin, mangostin, 4-hydroxyandrostenedione, 1,4,6-androstatrien-3,17-dione (ATD) and 4-androstene-3,6,17-trione (6-OXO).

In a particular embodiment of the composition of the invention, the ErbB3 inhibitor is selected from the group comprising IB4C3, 2D1D12, AMG888, 8B8, AV-203, B2B3-1, B2B3-2 and MM-121.

In a particular embodiment of the composition of the invention, the EGFR inhibitor is selected from the group comprising cetuximab, MM-151, Sym004, matuzumab, panitumumab, nimotuzumab, mAb 806, gefitinib, afatinib, lapatinib, canertinib, erlotinib HC, pelitinib, PKI-166, PD-158780, AG 1478, osrafenib and sunitinib.

In a particular embodiment of the composition of the invention, the mTOR inhibitor is selected from the group comprising rapamycin, sirolimus, temsirolimus, everolimus, ridaforolimus, KU-0063794 and WYE-354.

In the context of the invention, the composition can comprise as well a pharmaceutically acceptable vehicle, including additives, such as preservatives, excipients, loads, wetting agents, binding agents, disintegrating agents and buffers. These additives can be, for instance, magnesium and calcium carbonates, carboxymethyl cellulose, starches, sugars, gums, magnesium or calcium stearate, colouring matters, or flavouring agents. There is a high variety of pharmaceutically acceptable additives for pharmaceutical dosage forms and the selection of appropriate additives is a routine matter for the skilled in the art of pharmaceutical formulation. Excipients or vehicles preferred for use in this invention include sugars, starches, celluloses, gums, and proteins. In a particular embodiment, the pharmaceutical composition of the invention will be formulated in a solid (e.g., tablets, capsules, lozenges, granules, suppositories, sterile crystalline or amorphous solids that can be reconstituted to provide liquid forms etc.), liquid (for instance, solutions, suspensions, emulsions, elixirs, lotions, ointments, etc.) or semisolid (gels, ointments, creams, and similar) pharmaceutical dosage form.

### Compositions_for their use in the treatment of endocrine therapy-resistant breast cancer

In a further aspect, the invention relates to a composition as previously described for use in the treatment of breast cancer, wherein said breast cancer is resistant to endocrine therapy.

Alternatively, the invention relates to a composition as previously described in the manufacture of a medicament for the treatment of breast cancer, wherein said breast cancer is resistant to endocrine therapy. Alternatively, the invention relates to a method for the treatment of breast cancer that involves the administration of a therapeutically effective amount of a composition according to the invention to a subject suffering from breast cancer, wherein said breast cancer is resistant to endocrine therapy.

As a person skilled in the art understands, the composition of the invention for use in the treatment of breast cancer, wherein said breast cancer is resistant to endocrine therapy, will be formulated according to the administration route to be used.

The administration of the composition of the invention can be performed by different routes, for instance, intravenous, intraperitoneal, subcutaneous, intramuscular, topical, intradermal, intranasal or intrabronchial, and it can be administered locally or systemically or directly to the target site. A review of the different administration routes of active substances, of the excipients to be used and the manufacturing procedures can be found in Tratado de Farmacia Galénica, C. Faulli i Trillo, Luzán 5, S.A. de Ediciones, 1993 and in Remington's Pharmaceutical Sciences (A.R. Gennaro, Ed.), 20th edition, Williams & Wilkins PA, USA (2000).

The dosage regimen will be established by the physician and the clinical factors. As it is well known in medicine, the dosages depend on many factors, including the physical characteristics of the patient (age, size, sex), the administration route used, the severity of the disease, the particular compound used and the pharmacokinetic properties of the subject.

Methods to evaluate breast cancer resistance to a therapy, such as an endocrine therapy, as well as methods to monitor tumor response to a therapy, have been described in the context of the compounds of the invention for their use in the treatment of endocrine therapy-resistance breast cancer.

The invention is described below by the following examples, which must be considered as merely illustrative and in no case limiting of the scope of the present invention.

### EXAMPLES

The authors of the present invention have identified a 1-benzyl-3-(substituted aryl) condensed pyrazol, particularly YC-1, as a potent inhibitor of cell viability in long-term estrogen-deprived (LTED) MCF7 cells, a cellular model of acquired resistance to aromatase inhibitors in postmenopausal women. They have found also that YC-1 inhibits cell viability in cell line models of acquired resistance to fulvestrant, an ER antagonist, and to the raloxifene analog LY-117018, a selective estrogen receptor modulator.

### Materials and Methods

### Cell culture and viability assays

MCF7-LTED cells were established in phenol red-free Roswell Park Memorial Institute medium containing 10% dextran-coated charcoal-stripped fetal bovine serum. Cellular viability was evaluated using a standard methylthiazol tetrazolium (MTT; Sigma-Aldrich) assay. The results of these assays are expressed relative to vehicle treated controls and to the original time point.

### Chemical compound screen

MCF7 and MCF7-LTED cells were plated in 384-well microtiter plates and five compound dilutions (1 nM to 10 µM final concentration) from the LOPAC library (1,258 compounds, Sigma-Aldrich) were added to the cultures. Cell viability was assessed after 72 hours using MTT-based assays and the EnVision spectrofluorometer (Perkin-Elmer). The screen was performed in triplicate. The quality of the screen (Z'-factor > 0.5 for all screens) and the data was analyzed using the cell HTS2 module in the ScreenSaver database. The data was normalized between 0 and 1 using positive (1 µM phenylarsene oxide, Sigma-Aldrich) and negative (0.1% dimethyl sulfoxide, Merck) controls for cell viability. For hit selection, the difference between the normalize percentage inhibition (NPI) in MCF7 and MCF7-LTED cells was calculated by subtraction [ΔNPI = NPI(MCF7-LTED) - NPI(MCF7)] and the differentials were clustered with the MeV software package using the Cluster Affinity Search method with the Euclidean distance metric (threshold of 0.7).

### Example 1. Screening of chemical compounds reducing cell viability in an aromatase inhibitor-resistance breast cancer model

Acquired resistance to aromatase inhibitors in postmenopausal women can be modeled in long-term estrogen-deprived (LTED) MCF7 cells. Using this model, a cell-based chemical compound screen was carried out to identify potential therapeutic strategies that could prevent and/or overcome resistance. More than 1,200 compounds were assessed for their differential effect on the viability (as defined by MTT-based assays) of MCF7-LTED cells relative to the parental MCF7 cells. This screen identified 13 compounds showing higher inhibition in MCF7-LTED, while 6 compounds were found to have the opposite effect (Fig. 1A). Subsequent validation using independent cell cultures and compound solutions showed YC-1 (3-(5'-hydroxymethyl-2'-furyl)-1-benzylindazole) to be the most active as evaluated by the half maximal inhibitory concentration (IC₅₀): 4.9 µM and 131 µM for MCF7-LTED and MCF7 cells, respectively (Fig. 1B). BAY 41-2272, also displayed differences in cell viability, although of lesser magnitude (Fig. 1C). No differences were observed in the growth rate of the two cellular models when the cells were treated with Gefitinib (Fig. 1D).

### Example 2. Effect of YC-1 in estrogen receptor antagonist-resistant and SERM-resistant breast cancer models

Having identified the comparative inhibitory effect of YC-1 on the viability of MCF7-LTED cells, we evaluated the effect of this compound on a collection of breast cancer cell lines. Values of IC₅₀ < 10 µM were obtained for several cell lines (Table 1), notably the MCF7-LCC9 and MCF7-LY2 lines, which correspond to models of acquired resistance to fulvestrant and to the raloxifene analog LY-117018, respectively; these cell lines also showed cross-resistance to tamoxifen. For the remaining sensitive cell lines (as defined by an IC₅₀ < 10 µM threshold), no associations with ERα and/or progesterone receptor (PR) and/or human epidermal growth factor receptor 2 (HER2) were observed (Table 1).

**Table 1. YC-1 IC₅₀ (µM) values in breast cancer cell lines**

| Cell line | ERα | PR | HER2 | IC₅₀ |
|---|---|---|---|---|
| AU565 | - | - | + | 1.3* |
| BT474 | + | + | + | 319 |
| HCC1937 | - | - | - | 253 |
| MCF7 | + | + | -/+ | 131 |
| MCF7-HER2 | + | + | + | 187 |
| MCF7-LCC9 | + | ++ | - | 2.6* |
| MCF7-LTED | ++ | + | -/+ | 4.9* |
| MCF7-LY2 | + | ++ | - | 2.3* |
| MCF10A | - | - | - | 99 |
| MDA-MB-231 | - | - | - | 4,482 |
| MDA-MB-453 | - | - | - | 250 |
| MDA-MB-468 | - | - | - | 49 |
| SKBR3 | - | - | + | 1.6* |
| T47D | + | + | -/+ | 8.2* |
| ZR751 | + | - | -/+ | 0.3* |

| | | | | |
|---|---|---|---|---|
| * IC₅₀ < 10 µM | | | | |

To determine the possible-existence of a molecular phenotype characteristic of the sensitive cell lines, irrespective of growth factor receptor status, the inventors analyzed whole-genome expression data publicly-available for most of the lines. Using the GSEA tool, the sensitive cell lines showed over-expression of genes from the cell cycle pathway, while the insensitive-cells showed over-expression of genes from the ribosome pathway, among others. Increased dependence of the cell cycle has been previously noted in the response to anti-cancer therapies.

To determine the YC-1 mechanism of action that may be selective in models of acquired resistance, the subcellular localization of ERα was first examined. Although ERα levels and localization were altered by YC-1 treatment, no substantial differences were observed between MCF7-LTED and MCF7 cells. Subsequently, whole-genome expression data was obtained and analyzed for the basal and YC-1-exposed conditions in both cell lines. Consistent with the results described above, expression of the ribosome pathway clearly distinguished MCF7-LTED and MCF7 cells in basal conditions. Accordingly, when MCF7 cells were exposed to YC-1, the ribosome pathway was significantly altered, correlating with a decrease in phospho-serine 235/236 ribosomal S6 protein. In contrast, exposure of MCF7-LTED cells to YC-1 led to alteration of the cell cycle pathway and a decrease in a central positive regulator of the cell cycle, E2F1.

## Claims

1. A compound of formula (I) 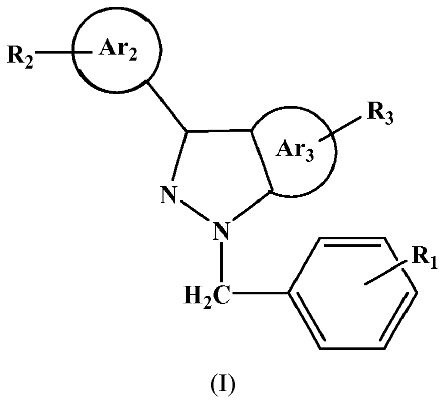 wherein
R₁ represents H, C₁₋₃ alkyl, halogen, or -OR, in which R represents H or C₁₋₃ alkyl; 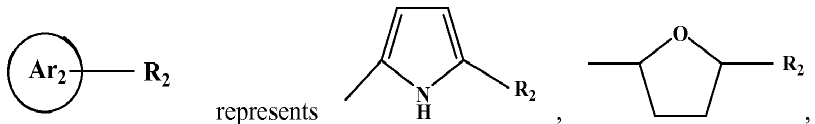 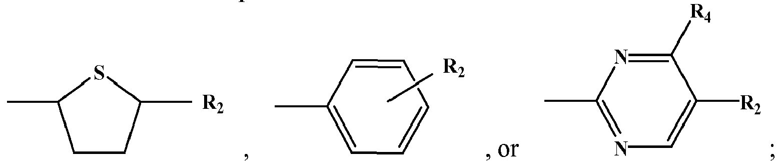 R₂ represents -COOR, -CH₂OR, H, C₁₋₃ alkyl, C₃₋₇ cycloalkyl, or halogen, in which R represents H or C₁₋₃ alkyl;
R₄ represents H, -NH₂, or 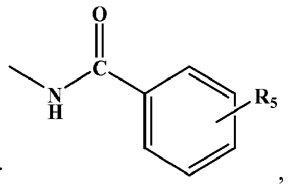 wherein R₅ is H or N₃, 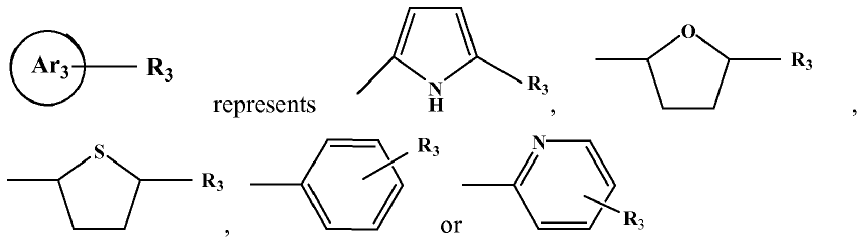 R₃ represents H, C₁₋₃ alkyl, halogen, or -OR, in which R represents H or C₁₋₃ alkyl; and pharmaceutically acceptable salts thereof, for use in the treatment of breast cancer, wherein said breast cancer is resistant to endocrine therapy.

2. A compound for use according to the preceding claim wherein
R₁ represents H, C₁₋₃ alkyl, halogen, or -OR, in which R represents H or C₁₋₃ alkyl; 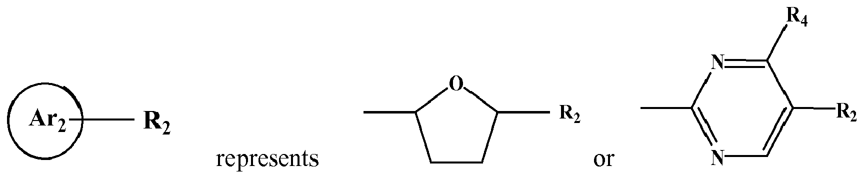 R₂ represents -COOR, -CH₂OR, H, C₁₋₃ alkyl, C₃₋₇ cycloalkyl, or halogen, in which R represents H or C₁₋₃ alkyl;
R₄ represents H, -NH₂, or 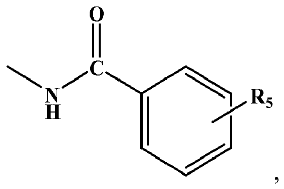 wherein R₅ is H or N₃, 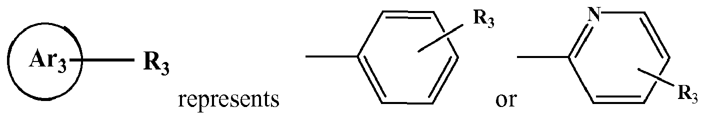 R₃ represents H, C₁₋₃ alkyl, halogen, or -OR, in which R represents H or C₁₋₃ alkyl.

3. A compound for use according to the preceding claim wherein
R₁ represents H, C₁₋₃ alkyl or halogen; 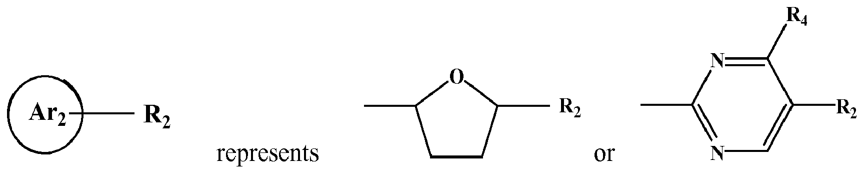 R₂ represents -COOR, -CH₂OR, H, C₁₋₃ alkyl, C₃₋₇ cycloalkyl, or halogen, in which R represents H or C₁₋₃ alkyl;
R₄ represents H, -NH₂, or 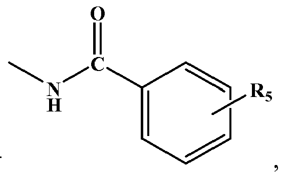 wherein R₅ is H or N₃, 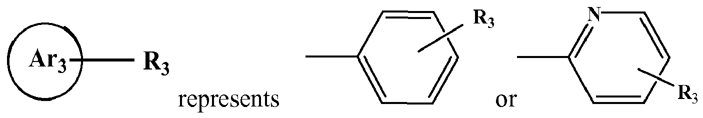 R₃ represents H, C₁₋₃ alkyl or halogen.

4. A compound for use according to any of the preceding claims, wherein the compound is selected from the group consisting of a compound having the formula 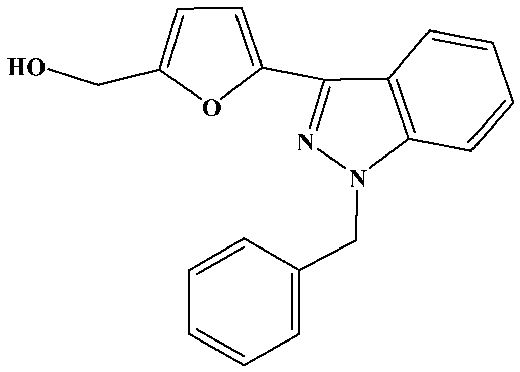 and a compound having the formula

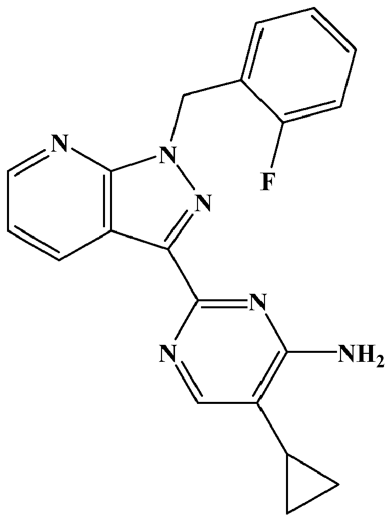

5. A compound for use according to any of the preceding claims wherein the breast cancer is resistant to endocrine therapy by acquired resistance to endocrine therapy.

6. A compound for use according to any of the preceding claims wherein the endocrine therapy is selected from the group consisting of an estrogen receptor antagonist, a selective estrogen receptor modulator (SERM) and an aromatase inhibitor.

7. A compound for use according to claim 6 wherein the estrogen receptor antagonist is fulvestrant.

8. A compound for use according to claim 6 wherein the selective estrogen receptor modulator is selected from the group comprising clomiphene, DT56a, ormeloxifene, raloxifene, tamoxifen, toremifene, lasofoxifene, afimoxifene, arzoxifene and bazedoxifene.

9. A compound for use according to claim 6 wherein the aromatase inhibitor is selected from the group consisting of sminoglutethimide, testolactone, anastrazole, letrozole, exemestane, vorozole, formestane, fadrozole, resveratrol, nicotine, myosmine, zinc, catechin, chalcones, apigenin, eriodictyol, isoliquiritigenin, mangostin, 4-hydroxyandrostenedione, 1,4,6-androstatrien-3,17-dione (ATD) and 4-androstene-3,6,17-trione (6-OXO).

10. A compound for use according to claim 10 wherein the aromatase inhibitor is selected from the group comprising anastrazole, letrozole and exemestane.

11. A composition comprising
i) a first compound of formula (I) 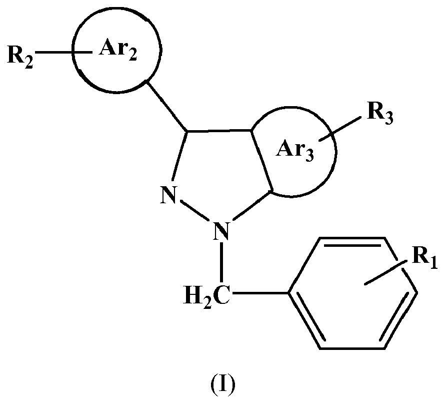 wherein
R₁ represents H, C₁₋₃ alkyl, halogen, or -OR, in which R represents H or C₁₋₃ alkyl; 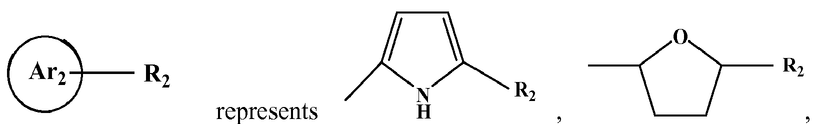 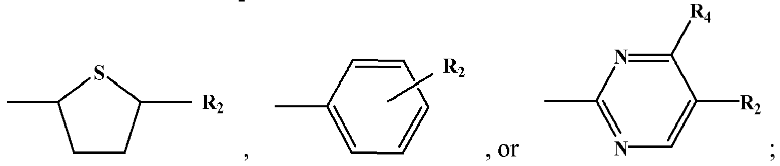 R₂ represents -COOR, -CH₂OR, H, C₁₋₃ alkyl, C₃₋₇ cycloalkyl, or halogen, in which R represents H or C₁₋₃ alkyl;
R₄ represents H, -NH₂, or 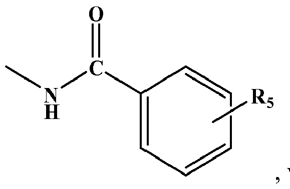 wherein R₅ is H or N₃, 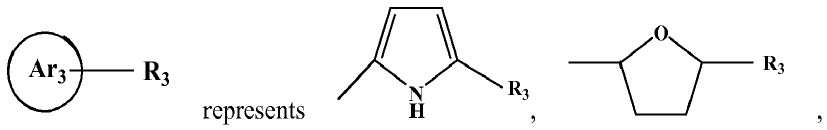 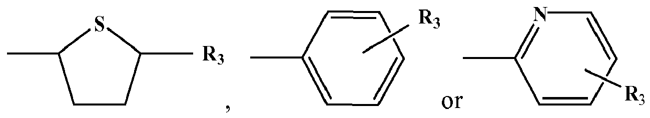 R₃ represents H, C₁₋₃ alkyl, halogen, or -OR, in which R represents H or C₁₋₃ alkyl; or any pharmaceutically acceptable salts thereof, and
ii) a second compound selected from the group comprising an estrogen receptor antagonist, a selective estrogen receptor modulator (SERM), an aromatase inhibitor, an ErbB3 inhibitor, an EGFR inhibitor and an mTOR inhibitor.

12. A composition according to the preceding claim wherein
R₁ represents H, C₁₋₃ alkyl, halogen, or -OR, in which R represents H or C₁₋₃ alkyl; 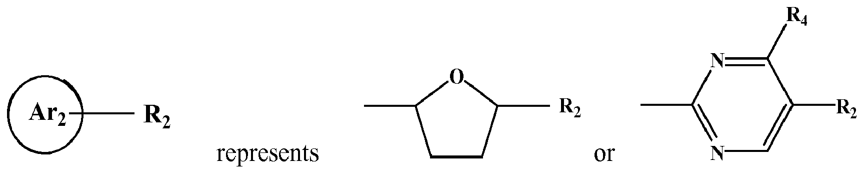 R₂ represents -COOR, -CH₂OR, H, C₁₋₃ alkyl, C₃₋₇ cycloalkyl, or halogen, in which R represents H or C₁₋₃ alkyl;
R₄ represents H, -NH₂, or 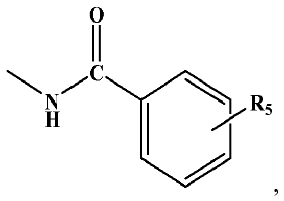 wherein R₅ is H or N₃, 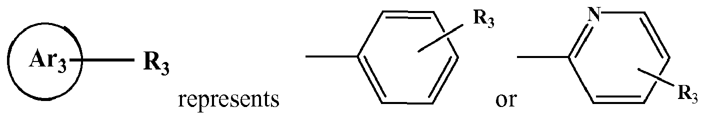 R₃ represents H, C₁₋₃ alkyl, halogen, or -OR, in which R represents H or C₁₋₃ alkyl.

13. A composition according to any of claims 11 or 12 wherein the first compound is selected from the compound having the formula 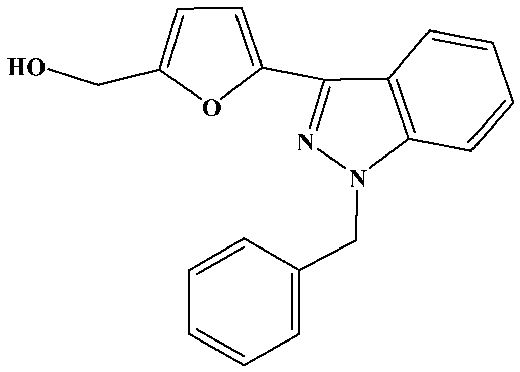 and a compound having the formula

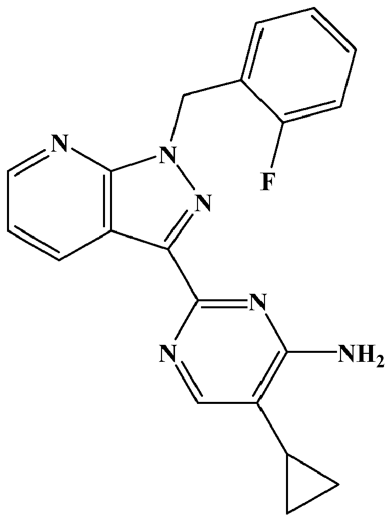

14. A composition according to any of claims 11 to 13 wherein
- the estrogen receptor antagonist is fulvestrant,
- the selective estrogen receptor modulator (SERM) is selected from the group comprising clomiphene, DT56a, ormeloxifene, raloxifene, tamoxifen, toremifene, lasofoxifene, afimoxifene, arzoxifene and bazedoxifene,
- the aromatase inhibitor is selected from the group comprising sminoglutethimide, testolactone, anastrazole, letrozole, exemestane, vorozole, formestane, fadrozole, resveratrol, nicotine, myosmine, zinc, catechin, chalcones, apigenin, eriodictyol, isoliquiritigenin, mangostin, 4-hydroxyandrostenedione, 1,4,6-androstatrien-3,17-dione (ATD) and 4-androstene-3,6,17-trione (6-OXO),
- the ErbB3 inhibitor is selected from the group comprising IB4C3, 2D1D12, AMG888, 8B8, AV-203, B2B3-1, B2B3-2 and MM-121,
- the EGFR inhibitor is selected from the group comprising cetuximab, MM-151, Sym004, matuzumab, panitumumab, nimotuzumab, mAb 806, gefitinib, afatinib, lapatinib, canertinib, erlotinib HC, pelitinib, PKI-166, PD-158780, AG 1478, osrafenib and sunitinib, or
- the mTOR inhibitor is selected from the group comprising rapamycin, sirolimus, temsirolimus, everolimus, ridaforolimus, KU-0063794 and WYE-354

15. A composition according any of claims 11 to 14 for use in the treatment of breast cancer, wherein said breast cancer is resistant to endocrine therapy.
